(19) Europäisches Patentamt / European Patent Office / Office européen des brevets

(11) **EP 4 261 826 B1**

(12) **EUROPEAN PATENT SPECIFICATION**

(45) Date of publication and mention
of the grant of the patent:
**05.03.2025 Bulletin 2025/10**

(21) Application number: **23167234.6**

(22) Date of filing: **10.04.2023**

(51) International Patent Classification (IPC):
**G11C 13/00** $^{(2006.01)}$    G06F 11/10 $^{(2006.01)}$
**G11C 29/04** $^{(2006.01)}$    H01J 49/00 $^{(2006.01)}$

(52) Cooperative Patent Classification (CPC):
**G11C 13/0019; G11C 13/004;** G06F 11/1048;
G11C 2029/0409; G11C 2029/0411; H01J 49/0036;
H01J 49/0045

(54) **SEQUENCING DATA-ENCODED PEPTIDES FROM TANDEM MASS SPECTRA**

SEQUENZIERUNG DATENKODIERTER PEPTIDE AUS TANDEM-MASSENSPEKTREN

SÉQUENÇAGE DE PEPTIDES CODÉS PAR DES DONNÉES À PARTIR DE SPECTRES DE MASSE EN TANDEM

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **11.04.2022 US 202263362757 P**

(43) Date of publication of application:
**18.10.2023 Bulletin 2023/42**

(73) Proprietor: **The HongKong Polytechnic University Hung Hom, Kowloon (HK)**

(72) Inventors:
• **YAO, Zhongping**
  **Hung Hom, Kowloon (HK)**
• **NG, Cheuk Chi**
  **Hung Hom, Kowloon (HK)**
• **LAU, Francis Chung Ming**
  **Hung Hom, Kowloon (HK)**
• **TAM, Wai Man**
  **Hung Hom, Kowloon (HK)**

(74) Representative: **Fabry, Bernd**
**IP2 Patentanwalts GmbH**
**Schlossstrasse 523-525**
**41238 Mönchengladbach (DE)**

(56) References cited:
**US-A1- 2002 102 610    US-A1- 2019 147 983**
**US-A1- 2021 090 688**

# EP 4 261 826 B1

**Description**

## LIST OF ABBREVIATIONS

| AAC | amino acid combination |
| DAG | directed acyclic graph |
| DNA | deoxyribonucleic acid |
| MS/MS | tandem mass spectrometry |
| $m/z$ | mass-to-charge |
| OFF | offset frequency function |
| RS | Reed-Solomon |
| TIC | total ion chromatogram |

## TECHNICAL FIELD

The present disclosure generally relates to sequencing of data-encoded peptides. Particularly, the present disclosure relates to a method of sequencing a data-encoded peptide from an experimental spectrum obtained in analyzing the data-encoded peptide by mass spectrometry or MS/MS.

## BACKGROUND

Data storage using peptides offer several advantages over using DNAs. Most notably, a higher storage density can be achieved by using peptides over using DNAs in that unnatural amino acids can also be used for forming the peptides such that an alphabet size for encoding digital data into amino acids is enlarged. Furthermore, data storage using the peptides enjoy a longer storage time than using DNAs due to the property that peptides are more durable than DNAs.

Peptide sequencing is an important element in decoding data stored in data-encoded peptides. Mass spectrometry, especially MS/MS, is particularly useful for peptide sequencing. US 11,315,023 B2 discloses techniques of sequencing data-encoded peptides from experimental spectra obtained in mass spectrometry or MS/MS. It is desirable to have improved techniques of sequencing the data-encoded peptides in mass spectrometry or MS/MS. US 2021/0090688 A1 describes methods and systems for storing digital data into peptide sequences and retrieving digital data from peptide sequences.

## SUMMARY

The invention is defined in independent claim 1 which provides for a computer-implemented method for sequencing a data-encoded peptide from an experimental spectrum. Further preferred embodiments are defined in the dependent claims.

## BRIEF DESCRIPTION OF THE DRAWINGS

FIG. **1** depicts a 4095 × 17 block of 3-bit symbols having a total number of 208845 (= 4095 × 17 × 3) bits as a first example of designing data carried on data-encoded peptides.

FIG. **2** depicts a 4095 × 19 block of 3-bit symbols having a total number of 98268 information bits, 12285 order-checking bits and 3 RS codes as a second example of designing data carried on data-encoded peptides.

FIG. **3** depicts a flowchart illustrating a method of two-stage sequencing in accordance with certain embodiments of the present disclosure.

FIG. **4** depicts a spectrum graph illustrating path finding under a graph theory model in accordance with certain embodiments of the present disclosure.

FIG. **5** depicts a flowchart illustrating a method of highest-intensity-tag based sequencing in accordance with certain embodiments of the present disclosure.

FIG. **6** depicts a conceptual diagram of a candidate sequence as determined by the highest-intensity-tag based sequencing method in accordance with certain embodiments of the present disclosure.

FIG. **7** depicts a flowchart illustrating a first method of finding prefix and suffix of the highest-intensity-based tag to form a possible candidate sequence, where the prefix and suffix are identified based on a maximum length of AACs in accordance with certain embodiments of the present disclosure.

FIG. **8** depicts a flowchart illustrating a second method of finding prefix and suffix of the highest-intensity-based tag to form a possible candidate sequence, where the prefix and suffix are identified based on a maximum length of AACs in accordance with certain embodiments of the present disclosure.

FIG. **9** depicts a flowchart illustrating a checking method for candidate sequences in accordance with embodiments of the disclosure.

FIG. **10** depicts a TIC of an example mixture of data-encoding peptides as obtained from an experiment.

FIG. **11** depicts a flowchart for illustrating a computer-implemented method for sequencing a data-encoded peptide from an experimental spectrum in accordance with an exemplary embodiment of the present disclosure.

FIG. **12** depicts a flowchart illustrating exemplary steps of processing raw data of experimental spectrum to generate preprocessed data.

Skilled artisans will appreciate that elements in the figures are illustrated for simplicity and clarity and have not necessarily been depicted to scale. For example, the dimensions of some of the elements in the illustrations, block diagrams or flowcharts may be exaggerated in respect to other elements to help to improve understanding of the present embodiments.

## DETAILED DESCRIPTION

The present disclosure is concerned with peptide sequencing for data-encoded peptides. Two related peptide sequencing methods are disclosed, namely, a two-stage sequencing method and a highest-intensity-tag based sequencing method.

Section A describes the technical terms and notations used in the present disclosure. Section B provides some examples of designing peptide sequences for data-encoded peptides. The resultant peptide sequence designs are occasionally referenced in explaining the two sequencing methods. The problem of sequencing data-encoded peptides is modelled in Section C. The two-stage sequencing method and the highest-intensity-tag based sequencing method are elaborated in Sections D and E, respectively. Section F provides a checking method based on the design of peptide sequences for verifying candidate sequences during generation of these sequences. Embodiments of the present disclosure are developed mainly based on the developments in Sections D-F.

### A. Technical Terms and Notations

The following terms and notations are used herein in the specification and appended claims.

"A peptide" is a physical chain of amino acids linked by peptide bonds.

"A data-encoded peptide" is a peptide whose constituent amino acids are purposely selected and positioned in the peptide in a meaningful manner for representing digital data.

"A peptide sequence" of a peptide is a digital string representing an ordered sequence of amino acid residues in the peptide. Thus, the ordered sequence specifies an order of assembly of constituent amino acids in forming the peptide.

"A head amino acid" is defined as an amino acid residue next to the N-terminus of a peptide. That is, the head amino acid is an N-terminal amino acid residue of the peptide.

"A tail amino acid" is defined as an amino acid residue next to the C-terminus of a peptide. That is, the tail amino acid is a C-terminal amino acid residue of the peptide.

The meanings of "b-ion", "y-ion" and other fragment ions due to cleaving peptide bonds of a peptide are commonly understood by those skilled in the art. It is intended that the present disclosure adopts commonly-accepted interpretation of b-ion, y-ion and other fragment ions obtained by breaking up peptide bonds of a peptide. A b-ion of a peptide is a charged fragment of the peptide after the peptide is split at a peptide bond between two constituent amino acids, where the b-ion contains the N-terminus of the peptide. A y-ion of a peptide is a charged fragment of the peptide after the peptide is split at a peptide bond between two constituent amino acids, where the y-ion contains the C-terminus of the peptide.

"A tag" is a partial sequence of an amino-acid sequence where the partial sequence is formed by consecutive amino acids.

"An AAC" is an order-independent composition of amino acids. Note that an order of assembly of constituent amino acids in an AAC needs not be known in modeling or initially establishing the AAC. Nonetheless, in peptide sequencing, it is often necessary to resolve the order of assembly for the AAC based on additional information on the AAC.

"A mass spectrum" is a histogram of intensity versus mass/charge ratio in a chemical sample analyzed by a mass spectrometer.

"An experimental spectrum" is a mass spectrum obtained in an experiment.

"Raw data of an experimental spectrum," are data of a histogram of intensity versus mass/charge ratio as originally obtained in analyzing a chemical sample by mass spectrometry. In practice, the originally obtained histogram is usually obtained from a mass spectrometer. In the present disclosure, however, it is intended that signal enhancement techniques (such as noise reduction algorithms) may be used in preparing the originally obtained histogram from physical measurement data obtained from the mass spectrometer.

"A match error" is defined as a mean error between observed mass values for amino acids retrieved from an experimental spectrum and actual mass values of the amino acids normalized by the corresponding observed mass values.

"A parent ion peak", also known as a molecular ion peak, in a mass spectrum is a peak that stems from a molecule that is electrically charged when the molecule is analyzed in a mass spectrometer without any fragmentations.

Denote $A = \{a_1, a_2, ..., a_K\}$ as a set of amino acids that are selectable to be used as a constituent amino acid in forming a data-encoded peptide, where K is the number of different amino acids in the set, and $a_i$, $i \in \{1, ..., K\}$, is an ith amino acid in $A$. In other words, A is an alphabet of amino acids for forming the data-encoded peptide.

Denote $g = \{g_1, g_2 ... , g_K\}$ as a mass set of amino acid residues that are selectable to be used in forming the data-encoded peptide, where $g_i$, $i \in \{1, ..., K\}$ is the mass of an amino acid residue corresponding to $a_i$.

Denote $P = \{P_1, P_2, ..., P_N\}$ as a peptide sequence of a peptide, where $N$ is the number of constituent amino acids that collectively form the peptide, and $P_i$, $i \in \{1, ..., N\}$, is an ith amino acid residue in the peptide sequence. Without causing confusion, the peptide is also denoted by $P$, and it is understood that the peptide referred to as $P$ is a peptide whose peptide sequence is $P$.

Denote m = $\{m_1, m_2, ... , m_N\}$ as a mass set of amino acid residues in the peptide $P$, where $m_i$, $i \in \{1, ... , N\}$, is the mass of $P_i$.

Denote $m_H$ as the mass of a hydrogen atom or a proton.

Denote $m_{OH}$ as the mass of a hydroxyl group.

Denote $m_{Ngroup}$ as the mass of an N-terminal functional group attached to a head amino acid of the peptide $P$. If $P$ has an unprotected N-terminus, the mass of the N-terminal functional group is equal to $m_H$.

Denote $m_{Cgroup}$ as the mass of a C-terminal functional group attached to a tail amino acid of the peptide $P$. If $P$ has an unprotected C-terminus, the mass of the C-terminal functional group is equal to $m_{OH}$.

Denote $m_{head}$ and $m_{tail}$ as the masses of the head amino acid and of the tail amino acid, respectively. The masses $m_{head}$ and $m_{tail}$ can be zero if there are no fixed amino acids.

Denote M as the mass of the peptide $P$. Note that $M$ is given by $M = m_{Ngroup} + m_{Cgroup} + \sum_{j=1}^{N} m_j$.

Denote $m_b = \{m_{b,1}, m_{b,2}, ... , m_{b,N+1}, m_{b,N+2}\}$ as the mass set of b-ions in the peptide P, where $m_{b,i+1} = m_{b,i} + m_i$ for $i \in \{1, ... , N+1\}$ with $m_{b,1} = m_{Ngroup} - m_H$. Note that: $m_{b,2} = (m_{Ngroup} - m_H) + m_{head}$; $m_{b,N} = M - m_H - m_{Cgroup} - m_{tail}$; $m_{b,N+1} = M - m_H - m_{Cgroup}$; and $m_{b,N+2} = M$.

Denote $m_y = \{m_{y,1}, m_{y,2}, ... , m_{y,N+1}, m_{y,N+2}\}$ as the mass set of y-ions in the peptide $P$, where $m_{y,i+1} = m_{y,i} - m_i$ for $i \in \{1, ..., N+1\}$ with $m_{y,1} = M - (m_{Ngroup} - m_H)$. Note that $m_y = M - m_b$. Also note that: $m_{y,2} = M - (m_{Ngroup} - m_H) - m_{head}$; $m_{y,N} = m_H + m_{Cgroup} + m_{tail}$; $m_{y,N+1} = m_H + m_{Cgroup}$; and $m_{y,N+2} = 0$.

Denote $\Delta = \{\Delta_1, \Delta_2 ... , \Delta_N\}$ as the mass difference set between a theoretical (mass) spectrum and an experimental (mass) spectrum, where $\Delta_i \in [-\delta, +\delta]$, $i \in \{1, ..., N\}$, and $\delta$ is a tolerance value.

Denote $T(P)$ as the theoretical spectrum generated by the peptide $P$.

Denote $S$ as a MS/MS spectrum obtained by tandem mass spectrometry.

Denote $L$ as the number of pairs of $m/z$ ratio and intensity representing the spectrum $S$.

Denote z = $\{z_1, z_1, ... , z_L\}$ as the set of charges for the spectrum $S$. Usually, $z_i$ is selected from 1, 2 and 3.

Denote $(m/z) = \{(m/z)_1, (m/z)_2, ... , (m/z)_L\}$ as a set of mass/charge ratios for the spectrum S.

Denote $\rho$ as the number of subsets in the spectrum $S$. In each subset, all $(m/z)$ ratios are isotopes of a particular fragment, with a charge value equaling the inverse of difference between consecutive mass/charge ratios.

Denote $M_{out}$ as the mass output of a mass spectrometer corresponding to the highest intensity. The mass output $M_{out}$ may be a monoisotopic peak, or the peak that follows (with mass +1.007276 Da).

Denote $G_i$, $i \in \{1,2, ..., \rho\}$, as an ith subset in the spectrum $S$.

Denote $m'_i$ as a monoisotopic mass for the subset $G_i$. Note that $m'_i$ can be calculated by $m'_i = (m/z)_{i,0} z'_{i,0} - m_H z'_{i,0}$, where $(m/z)_{i,0}$ is the lowest value in the subset $G_i$, and $z'_{i,0}$ is the corresponding charge for $(m/z)_{i,0}$,

Denote $m'_b = \{m'_{b,1}, m'_{b,2}, ... , m'_{b,L}\}$ as the mass set of putative b-ions for the spectrum $S$, where $m'_{b,i} = (m/z)_i z_i - m_H z_i$, $i = 1, ... , L$.

Denote $m'_y = \{m'_{y,1}, m'_{y,2}, ... , m'_{y,L}\}$ as the equivalent b-ion mass set of putative y-ions for the spectrum $S$, where $m'_{y,i} = M - [(m/z)_i z_i - m_H z_i]$, $i = 1, ... , L$.

Denote $I = \{I_1, I_2, ... , I_L\}$ as a set of intensities for the spectrum $S$, where the intensity $I_i$ corresponds to $(m/z)_i$ for each $i$, $i = 1,2, ..., L$.

Denote $J$ as the ranking of the intensity.

Denote $m'_{B,J}$ as the mass of the putative b-ion with the $J$th highest intensity for the spectrum S.

Denote $m'_{Y,J}$ as the equivalent b-ion mass of the putative y-ion with the $J$th highest intensity for the spectrum $S$.

Denote $J_{max}$ as the maximum number of higher-ranking-intensity masses allowed to be a start point in finding a tag.

Denote $n$ as the number of candidate sequences.

Denote $W$ as the number of masses with higher ranking intensity used for a tag-finding scheme.

Denote $V$ as the maximum number of iterations in a highest-intensity-tag based sequencing method.

Denote $L_{AAC}$ as the length of an AAC.

Denote $L_{p1}$ and $L_{p2}$ as two maximum lengths of AACs used for limiting a length of each AAC in a prefix.

Denote $L_{s1}$ and $L_{s2}$ as two maximum lengths of AACs used for limiting a length of each AAC in a suffix.

Denote $P_L$ as the position of the last amino acid "L" in the peptide sequence $P$.

Denote $P_G$ as the position of the first amino acid "G" in the peptide sequence $P$.

Denote $L'$ as the number of symbols in a row for a $4095 \times 17$ block or for a $4095 \times 19$ block. Note that $L' = N - 2$.

Denote $Seq$ as the index of a sequence in the block, where $Seq$ ranges from 1 to 4095.

Denote $S_1, S_2, ... , S_{L'}$ be $L'$ symbols taken from the block and encoded in the peptide $P$ (excluding the head and tail amino acids).

Denote $Q_{i,j}$ as an order-checking bit used to protect the order of symbols $S_i$ and $S_j$, where $(i,j)$ is selected from $(1,2)$, $(2,3)$ and $(L' - 1, L')$. For details of order-checking bits, see US 11,315,023 B2.

Denote $A_1, A_2, A_3$ and $A_4$ as the address symbols $S_6, S_7, S_8$ and $S_9$, respectively.

Denote $n_i$ as the code length of an $i$th RS code.

Denote $k_i$ as the number of 12-bit information symbols for the $i$th RS code.

Denote $R$ as an overall code rate.

One-letter symbols are occasionally used to represent different amino acids in, e.g., describing a peptide's structure. Amino acids and their respective one-letter symbols (enclosed in paratheses) are listed as follows: Alanine (A); Arginine (R); Asparagine (N); Aspartate (D); Cysteine (C); Glutamine (Q); Glutamate (E); Glycine (G); Histidine (H); Isoleucine (I); Leucine (L); Lysine (K); Methionine (M); Phenylalanine (F); Proline (P); Serine (S); Threonine (T); Tryptophan (W); Tyrosine (Y); Valine (V); Selenocysteine (U); and Pyrrolysine (O).

## B. Design of Data-EncodedPeptides

In the design of the fixed-length sequences, each peptide sequence has $L'$ ($L'$ = 17) 3-bit symbols, which are denoted as $S_1, S_2, ... , S_{17}$. We make the assumptions that: (i) 15%, 10% and 25%, respectively, of the 4-symbol sequences $\{S_1, S_2, S_3, S_4\}$, $\{S_{10}, S_{11}, S_{12}, S_{13}\}$ and $\{S_{14}, S_{15}, S_{16}, S_{17}\}$ cannot be recovered correctly for the length-17 symbol sequence $\{S_1, S_2, ... , S_{17}\}$; and (ii) there are three ambiguous orders of symbols, which are the order of $S_1$ and $S_2$, the order of $S_2$ and $S_3$, and the order of $S_{16}$ and $S_{17}$.

Then, we propose an error-correction method based on the RS code. The method uses: (i) three RS codes to recover the original data even when any arbitrary 15% of 4-symbol sequences $\{S_1 S_2 S_3 S_4\}$, any arbitrary 10% of 4-symbol sequences $\{S_{10} S_{11} S_{12} S_{13}\}$, and any arbitrary 25% of 4-symbol sequences $\{S_{14} S_{15} S_{16} S_{17}\}$, cannot be recovered correctly, and (ii) three order-checking bits for the order of $S_1$ and $S_2$, the order of $S_2$ and $S_3$ and the order of $S_{16}$ and $S_{17}$ in each peptide sequence, respectively.

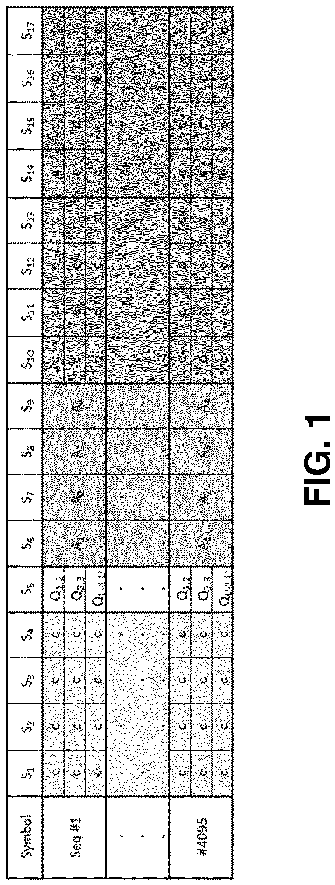

As shown in FIG. **1**, a $4095 \times 17$ block of 3-bit symbols is first constructed which has a total of 208845 (= $4095 \times 17 \times 3$) bits. In each row of the block, there are 17 symbols (i.e. $S_1, S_2, ... , S_{17}$) to represent a 17-mer data-encoded peptide. Four of the symbols, namely $S_6, S_7, S_8$ and $S_9$, are defined by $A_1, A_2, A_3$ and $A_4$, respectively. They serve as the address of the peptide sequence and assume one of the following values: 0000 (in octal form), 0001, 0002, ..., 0007, 0010, ..., 0777, 1000, ..., 7775, 7776. The three order-checking bits carried in $S_5$ are denoted as $Q_{1,2}$, $Q_{2,3}$ and $Q_{16,17}$, which are used to denote the order of $S_1$ and $S_2$, the order of $S_2$ and $S_3$ and the order of $S_{16}$ and $S_{17}$, respectively. The order-checking bit $Q_{i,j}$ is "1" if the value of $S_i$ is larger than that of $S_j$; otherwise, the order-checking bit is "0". The remaining 12 symbols of each row, i.e. $S_1, S_2, ..., S_5, S_{10}, ... , S_{17}$, can then be used to restore the coded bits c, including the information bits and parity bits of the error-correction codes.

Next, we assume that 15%, 10% and 25%, respectively, of the 4-symbol sequences $\{S_1 S_2 S_3 S_4\}$, $\{S_{10} S_{11} S_{12} S_{13}\}$ and $\{S_{14} S_{15} S_{16} S_{17}\}$ cannot be recovered correctly. We then use three different $(n_i, k_i)$ RS codes for these three partial sequences, where $n_i$ is the code length of the ith RS code and $k_i$ is the number of 12-bit information symbols for the ith RS code. It is assumed that $n_i$ takes the same value 4095 for $i$ = 1, 2 and 3, i.e. $n_1 = n_2 = n_3 = 4095$, while $k_1 = 2867, k_2 = 3275$ and $k_3 = 2047$ for the 1st, 2nd and 3rd RS codes, respectively. The resultant (4095, 2867) RS code (denoted as RS1), (4095, 3275) RS code (denoted as RS2) and (4095, 2047) RS code (denoted as RS3) can correct up to 614, 410 and 1024 12-bit symbol errors, respectively. For the whole block, there are at most $(k_1 + k_2 + k_3) \times 12 = 98268$ bits that can be used as the data bits, and the maximum overall code rate R of the block is given by $98268 / (4095 \times 17 \times 3) = 0.4705$.

For our dataset $A$, the number of information bits is 96224 in which there are 47965 bits of "0" and 48259 bits of "1". Since each $4095 \times 17$ block can contain up to 98268 information bits, we fill the remaining 98268 - 96224 = 2044 bit positions in each block with "0" and "1" with equal probability. Then these 98268 bits are divided into three parts. The first part is used to fill the symbols $\{S_1S_2S_3S_4\}$ in the row sequences ranging from $(n_1 - k_1 + 1)$ to $n_1$, the second part to fill the symbols $\{S_{10}S_{11}S_{12}S_{13}\}$ in the row sequences ranging from $(n_2 - k_2 + 1)$ to $n_2$, and the third part to fill the symbols $\{S_{14}S_{15}S_{16}S_{17}\}$ in the row sequences ranging from $(n_3 - k_3 + 1)$ to $n_3$. In the encoding process, RS1 uses the bits in the first part to generate the coded symbols to fill $\{S_1S_2S_3S_4\}$ in the row sequences ranging from 1 to $(n_1 - k_1)$; RS2 uses the bits in the second part to generate the coded symbols to fill $\{S_{10}S_{11}S_{12}S_{13}\}$ in the row sequences ranging from 1 to $(n_2 - k_2)$; RS3 uses the bits in the third part to generate the coded symbols to fill $\{S_{14}S_{15}S_{16}S_{17}\}$ in the row sequences ranging from 1 to $(n_3 - k_3)$.

Finally, we count the number of 3-bit symbols equating 000, 001, ..., 111, (or denoted by 0 to 7) in the block. The numbers for symbols 000, 001, ..., 111, are 8294, 8380, 8927, 9181, 8831, 8754, 8671 and 8577, respectively. The actual code rate R for our dataset $A$ is then given by 96224 / $(4095 \times 17 \times 3)$ = 0.4607.

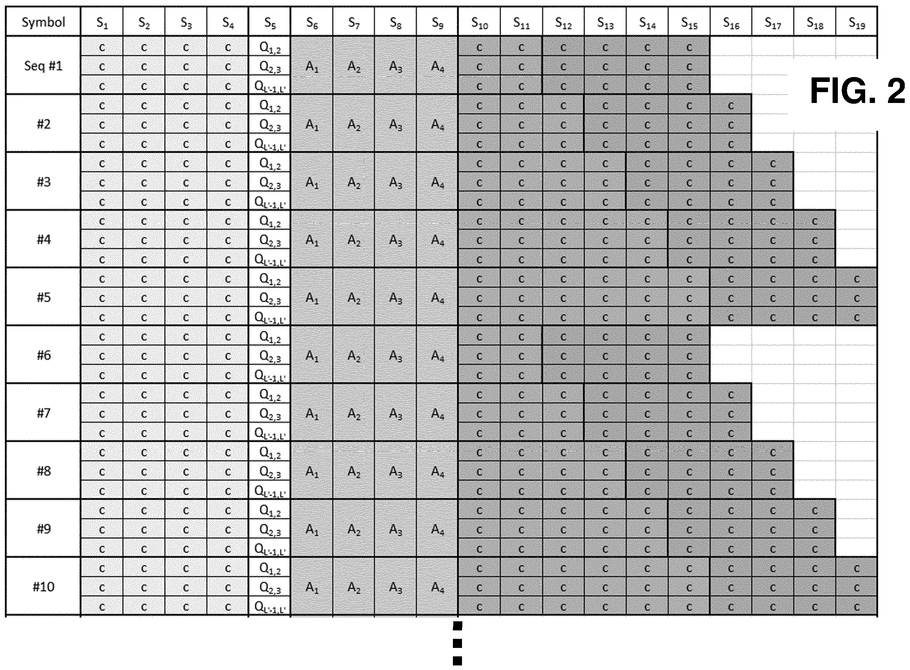

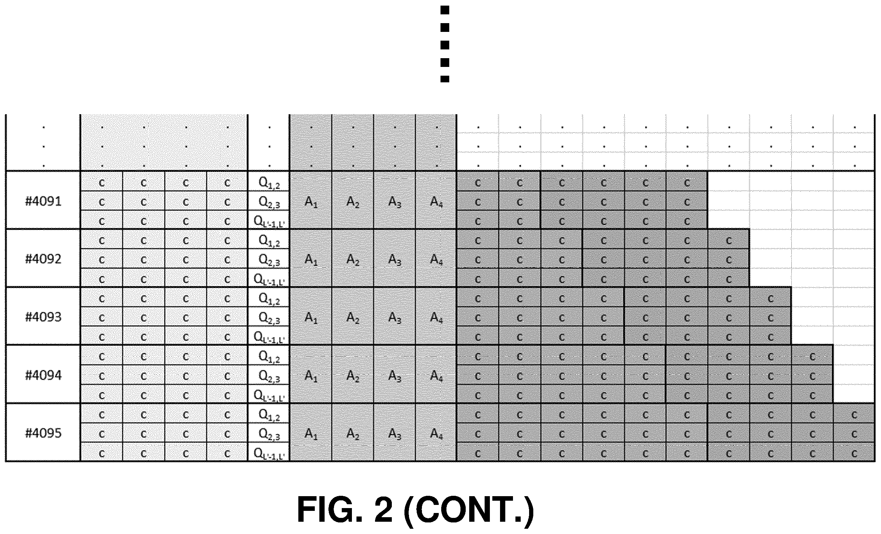

Now, we consider the case that the lengths of the sequences vary and we assume one of the following values: 15, 16, 17, 18, and 19. Then we construct the $4095 \times 19$ block shown in FIG. **2.** We regard every 5 sequences with lengths 15, 16, 17, 18, and 19 as a group. The length $L'_j$ for the $j$th sequence in each group can be evaluated by $L'_j$ = 15 + mod(Seq - 1,5), where mod represents the modulo operation, and $Seq$ is the index of the sequence in the block and ranges from 1 to 4095. Since $Seq$ and the address sequence $\{A_1A_2A_3A_4\}$ are one-to-one mapping with $Seq = A_1 \times 8^3 + A_2 \times 8^2 + A_3 \times 8^1 + A_4 + 1$, a length checking based on the address can be used to determine the correctness of the sequence.

Let $S_i^{(j)}$ be the ith symbol of the $j$th sequence in each group. As can be observed in FIG. **1,** we can transform the $4095 \times 17$ block to the $4095 \times 19$ block by: (i) moving $S_{10}^{(1)}$ and $S_{11}^{(1)}$ of Sequence 1 and inserting them before $S_{10}^{(5)}$ of Sequence 5; and moving $S_{10}^{(2)}$ of Sequence 2 and inserting it before $S_{10}^{(4)}$ of Sequence 4.

Since the variable-length sequences shown in FIG. **2** are constructed by moving the symbols in the fixed-length sequences shown in FIG. **1,** the overall code rate $R$ of the $4095 \times 19$ block in FIG. **2** is the same as that of the $4095 \times 17$ block in FIG. **1.**

Next, we map the 3-bit symbols (symbols from "0" to "7" corresponding to the three bits from 000 to 111) in the $4095 \times 17$ block into 8 amino acids (Y, T, E, A, S, V, G and F), and obtain the 4095 sequences with "G" corresponding to Symbol "6".

Moreover, if mass conflict occurs, that is, the mass $M$ of a sequence is within the tolerance range (25ppm) of other sequences, then the amino acid "G" is replaced by an unused amino acid "L". This process starts from the first "G" near the head amino acid. After the replacement of only one "G", if the updated mass $M$ still falls in the tolerance range of other sequences, the replacement process continues by shifting to the next "G" of the original sequence. After this process is performed, the sequences consist of 9 possible amino acids with both "G" and "L" corresponding to Symbol "6". Since all amino acids of "L" should be presented before "G", the order of "G" and "L" can be used to distinguish the validation of an estimated peptide sequence after peptide sequencing. The effect of this replacement can be seen from an example dataset with 4095 data-encoded peptides, with fixed N-terminal amino acid H and fixed C-terminal amino acid R, and with the amino acids in between encoded using the mapping of the 3-bit symbols into the 8 amino acids as described above. Before replacement, the number of data-encoded peptides with mass conflict was 3630, and there were 732 groups of data-encoded peptides where all data-encoded peptides in each group have the same $M$ within the tolerance range. After replacement, the number of data-encoded peptides with mass conflict was dropped to 2746, and there were 619 groups of data-encoded peptides where all data-encoded peptides in each group have the same $M$ within the tolerance range. It reduces the problem where two or more isobaric peptides co-elute, thus reducing the number of overlapping MS/MS spectra that interfere sequencing.

### C. Peptide Sequencing Problem

Generally, under the conditions of using the whole set of 20 natural amino acids and the existing peptides with natural amino acids, most of the existing sequencing algorithms, including Sherenga, PepNovo, NSNovo, pNovo, uniNovo, NovoHCD and Novor, rely on training by the properties of the data. For example, the OFFs introduced by V. DANČÍK et al. (*"De Novo* Peptide Sequencing via Tandem Mass Spectrometry, *Journal of Computational Biology,* vol. 6, no. 3/4, 1999, pp. 327-342") are first empirically derived by counting the occurrence frequencies of different ion types and feature types in the training data. After candidate paths are obtained by using the DAG model and the dynamic programming for Sherenga, pNovo and uniNovo, the OFFs are used as reference for the scoring of these paths.

In peptide-based data storage, the amino acids are generated by any combinations of bits "0" and "1" and hence the peptide sequences are completely random. Therefore, no suitable training sequences are available for existing sequencing algorithms. Also, peptide-based data storage may use some or all of the natural amino acids depending on the design of peptides. In addition, unnatural amino acids can be used.

A constraint for the dynamic algorithm is that no training spectra are provided for the sequencing of the digital data bearing peptides. Accordingly, de novo sequencing based on the graph model is utilized for determining peptide sequences. In the graph model, the MS/MS spectrum is represented by a DAG, called a spectrum graph. The peaks of the spectrum can be taken as vertices, while an edge is added between two vertices when the mass gap between two peaks is equal to the mass of an amino acid. The objective of dynamic programming is to find the longest path (or the best path) in the graph starting from the head vertex to the tail vertex. An alternative approach to identify the sequence is to start with the middle part of the MS/MS spectrum. For example, the sequence tagging method first infers a partial sequence called as a tag, and then finds the whole sequence that can match the tag. In the tag-based method, tags are first found from the MS/MS spectrum based on some scoring schemes. The inference of the sequence then relies on peptide comparison using the database search method or on extending the valid path of the tag in the middle position of the path using the de novo sequencing.

The peptide sequencing problem is outlined in Table **1,** with knowledge of information of the amino acids used, the spectrum $S$, the mass of the whole sequence, and the head and tail amino acids. The sequencing problem is to find the peptide $P$, whose theoretical spectrum $T(P)$ best matches the experimental spectrum S. In many practical situations, the length $N$ of the peptide sequence is fixed and known. Thus, candidate peptides of length not equal to $N$ are discarded. Two sequencing methods, namely a two-stage sequencing method and a highest-intensity-tag based sequencing method, are disclosed herein. For both methods, the sequences are estimated by first inferring a partial sequence with a small amount of reliable information and then finding the missing part of the sequence with less reliable data or the raw data. This approach gives an advantage of improving the speed of sequencing by generating a fewer number of candidates. Moreover, due to the introduction of the tag, the highest-intensity-tag based sequencing method is more effective in rejecting unlikely candidates.

Table **1.** Description of the peptide sequencing problem.

| Input: | Set $A$ of amino acids. |
|---|---|
| | Mass set $g$ for the amino acids in set $A$. |
| | Experimental spectrum $S$. |
| | Set ($m/z$) of mass/charge ratios of the spectrum S. |
| | Set $I$ of intensity of spectrum S. |
| | Length $N$ of the peptide sequence. |
| | Head and tail amino acids used for the peptide. |
| | Mass $M$ of the whole sequence. |
| Output: | Peptide sequence $P$ of length $N$. |
| Problem: | Estimate a peptide $P$ that most likely generates the experimental spectrum $S$. |

*D. Peptide Sequencing: Two-stage Sequencing Method*

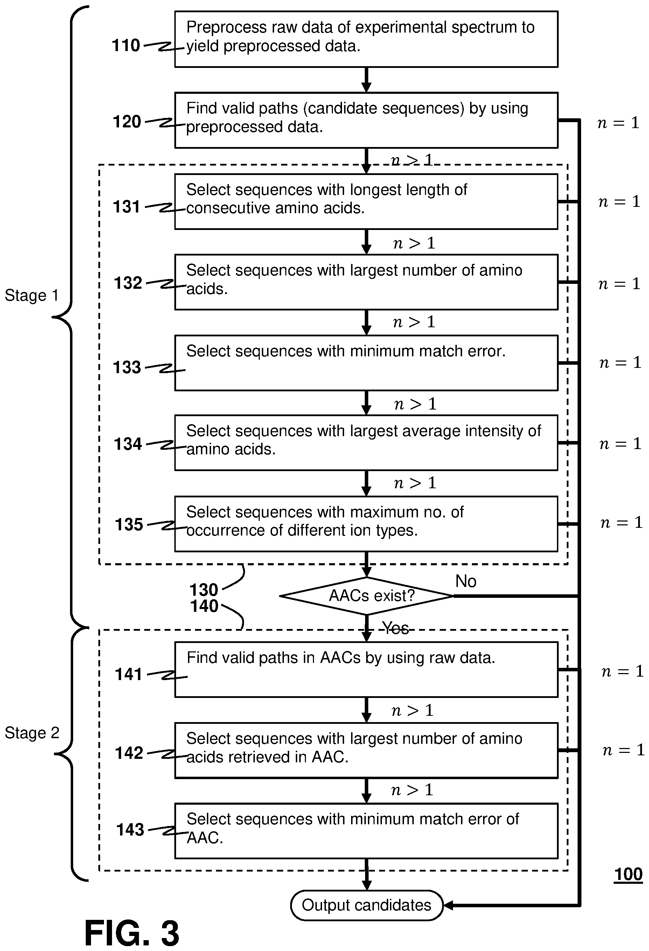

FIG. **3** shows a flowchart of exemplary steps for illustrating a method **100** of two-stage sequencing. Four steps are involved in the two-stage sequencing method **100**: preprocessing, candidate sequence generation, sequence selection, and candidate refining. As shown in FIG. **3**, steps **110, 120** and **130** belong to the first stage, i.e. Stage 1, while step **140** is processed in the second stage, i.e. Stage 2. In Stage 1 of the two-stage sequencing method **100**, a partial sequence is inferred by using the preprocessed data after the step **110** is performed. In Stage 2, the remaining part of the sequence is determined based on the raw data.

In the step **110,** preprocessing is performed. The goal of the preprocessing is twofold. First, it is to remove some uninterpretable peaks caused by noise and uncertainty. Second, it is to convert the mass/charge ratio set ($m/z$) to the corresponding mass sets $m'_b$ and $m'_y$. Given a set of mass/charge ($m/z$) ratios at which signal peaks are present in the experimental spectrum $S$, these ratios are divided into $\rho$ subsets $G_1, G_2, ... , G_\rho$, where in each subset $G_i, i \in \{1,2, ..., \rho\}$, all ($m/z$) ratios are isotopes of a particular fragment having the same chemical composition. Furthermore, the isotopes in each subset have the same charge state, which is represented by a charge of positive integer value (usually 1, 2 or 3). For each subset $G_i$, a monoisotopic mass $m'_i$ is calculated by $m'_i = (m/z)_{i,0} z'_{i,0} - m\mathrm{H}z'_{i,0}, i = 1,2, ..., \rho$, where $(m/z)_{i,0}$ is the lowest value in this subset, and $z'_{i,0}$ is the corresponding charge for $(m/z)_{i,0}$. These $m'_i$ values are then distributed between the mass sets $m'_b$ and $m'_y$. In certain embodiments, one of the criteria of distribution is based on the intensities corresponding to the $m'_i$ values. In certain embodiments, one of the criteria of distribution is the isotopic pattern of the ($m/z$) ratios in $G_i$. In certain embodiments, one of the criteria of distribution is based on the fact that if $m'_i$ is in $m'_{b,i}$, then there is a corresponding $m'_j$ in $m'_{y,j}$ where $m'_j = M - m'_i$. In certain embodiments, the distribution is determined real-time in step **120** to be described later.

In certain embodiments, only those data with typical property of charges are reserved as the preprocessed data such that the preprocessed data may be more reliable than the raw data. However, in the event of the incomplete or ambiguous data (e.g., data lacking charge property), some useful mass values may be discarded in the preprocessing based on the abovementioned criteria. Hence, in the case when missing/uncertain elements of the sequence exist in Stage 1, the raw data may be considered in Stage 2.

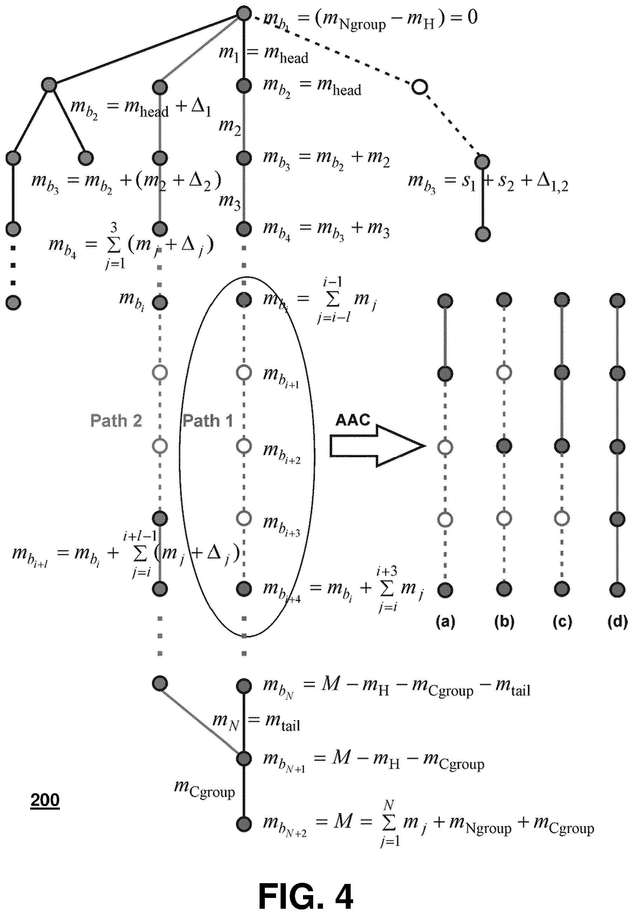

In the step **120,** the preprocessed data from the step **110** are used to find valid paths (sequences), and the number $n$ of candidate sequences is counted. Refer to FIG. **4,** which depicts a spectrum graph **200** as an example for illustrating the present disclosure. Suppose that $m_{\text{Ngroup}} = m_H$ and $m_{\text{Cgroup}} = m_{OH}$. The graph theory model is used to find the candidate paths (valid paths) each of which starts with a head mass $m_{\text{head}}$ and ends with mass $M - m_H - m_{\text{Cgroup}} - m_{\text{tail}}$ in a DAG. Since the masses obtained by mass/charge ratio set ($m/z$) are likely created by b-ion, y-ion, or both b-ion and y-ion. A single mass set ($m'_{b,i}$ or $m'_{y,i}$) or both mass sets ($m'_{b,i}$ and $m'_{y,i}$) can be considered in the path-finding algorithm. In the graph model, the mass of the fragment ion can be represented by a vertex. If the mass gap between two fragment ions equals the mass of any amino acid, then an edge is added between these two vertices. As shown in FIG. **4,** the tree can be expanded edge by edge. If the set of vertices is complete for the correct path, the sequencing problem can be reduced to finding the longest path in the graph. The path should include both the head and the tail vertices. Moreover, only those paths ending with mass $M$ are taken as candidates. In the example of FIG. **4,** only Path 1 and Path 2 are candidate paths.

The selection of suitable mass set(s) for peptide sequencing is strongly based on the peptide design. In one embodiment, F is the head (fixed amino acid at the N-terminus), R or K is the tail (fixed amino acid at the C-terminus) and no P or other basic amino acids in the middle. Previous experimental data shows that the parent ion peak with charge 2 is the strongest peak while the one with charge 3 is very weak, and the y-ions are much stronger than the b-ions when the charge 2 peak were selected for MS/MS. In this case, only the MS/MS spectra from the charge 2 peak would be analysed. Furthermore, using the masses of y-ions would be sufficient to find all sequences and thus only y-ions are used. In another embodiment, H is the head, R is the tail and no P or other basic amino acids in the middle. In this case, the charge 3 peak may be much stronger than, or as strong as the charge 2 peak. The MS/MS spectra of one or both parent peaks would be analysed. When the charge is 2, using the masses of y-ions is sufficient to find all sequences and thus only y-ions are used. However, when the charge is 3, both y- and b-ions are used. In another embodiment, the head and tails are not basic amino acids, the charge is usually 2, and both y- and b-ions would be used.

Due to the imperfect fragmentation in MS/MS characterizations, two and three missing ions are often observed in a sequence. Under the disclosed model of the peptide as considered, in order to ensure that the paths starting from the head can be extended to the tail, the number of missing amino acids is assumed to be up to $L_{AAC}$ in Stage 1. Starting from the mass with $m_{b,1} = 0$, firstly, an attempt is made to find the mass of the head amino acid, $m_{b,2} = m_{\text{head}} + \Delta_1$. Next, the mass $m_{b,i+1}$ is found by using the preprocessed data such that the mass gap between the current and the next vertices is approximate to mass $s_i$ or mass summation $\sum_{v=i}^{i+l=1} s_v$ (in which $l \leq L_{AAC}$) of up to $L_{AAC}$ amino acids with mass $s_v \in g$ (for $v = 1,2, \ldots, N$), i.e. $m_{b,i+1} = m_{b,i} + (s_i + \Delta_i)$ for two consecutive vertices $i$ and $i + 1$, or $m_{b,i+l} = m_{b,i} + (\sum_{v=i}^{i+l-1} s_v + \Delta_{i,i+l})$ (where $\Delta_{i,i+l} \in [-l\delta, +l\delta]$ for a length-$l$ tag from Vertex i to Vertex (i + l). Refer to FIG. **4.** Suppose that Path 2 is the correct path but with experimental masses, $m_{b,1} = 0$, $m_{b,i+1} = m_{b,i} + (m_i + \Delta_i)$ for two consecutive vertices, or $m_{b,i+l} = m_{b,i} + \sum_{j=i}^{i+l-1}(m_j + \Delta_j)$ for a length-$l$ tag. If Path 1 is the correct path with theoretical masses, then $m_{b,i+1} = m_{b,i} + m_i$ with $m_{b,1} = m_{\text{Ngroup}} - m_H$, $m_{b,2} = (m_{\text{Ngroup}} - m_H) + m_{\text{head}}, \ldots , m_{b,N} = M - m_H - m_{\text{Cgroup}} - m_{\text{tail}}$, $m_{b,N+1} = M - m_H - m_{\text{Cgroup}}$, and $m_{b,N+2} = M$. Refer to FIG. **4.** If the mass gap between two vertices equals the mass of an amino acid, then a solid edge is added. On the other hand, if the mass gap equals the mass summation of two or more amino acids, then the dashed edges are added with hollow circles representing the missing vertices.

In the step **130**, the effects of the following five factors are jointly considered when arriving at the score of a sequence candidate from step **131** to step **135**: length of consecutive amino acids retrieved (step **131**), number of amino acids retrieved (step **132),** match error (step **133),** average intensity of amino acids retrieved (step **134),** and number of occurrences for different ion types with different offsets (step **135**). The sequences with the longest length of consecutive amino acids retrieved are first selected (step **131**). Among the selected sequences, the sequences with the largest number of amino acids retrieved are then selected (step **132).** For the sequences with equal length of consecutive amino acids retrieved together with equal number of amino acids retrieved, the match error is evaluated. As mentioned above, the match error is a mean error between observed mass values for amino acids retrieved from the experimental spectrum and actual mass values of the amino acids normalized by the corresponding observed mass values (step **133**). If there are more-than-one sequences having an identical match error, the average intensity of amino acids retrieved is further calculated and a higher score is given to the sequence with a larger average intensity value (step **134).** In addition, multiple ion types are usually considered as important factors in inferring an amino acid. It means that a mass value may correspond to different types of ions in the spectrum. Generally, the higher number of occurrences for different ion-types of an amino acid is, the more likely the amino acid is correct. Therefore, for the sequences with equal score after the aforementioned evaluations of the steps **131-134** are performed, the number of occurrences for different ion-types is counted to determine the sequence (step **135).** The mass offset sets for the N-terminal a-ion, b-ion and c-ion type sets, i.e. {a, a-$H_2O$, a-$NH_3$, a-$NH_3$-$H_2O$}, {b, b-$H_2O$, b-$H_2O$-$H_2O$, b-$NH_3$, b-$NH_3$-$H_2O$}, and {c, c-$H_2O$, c-$H_2O$-$H_2O$, c-$NH_3$, c-$NH_3$-$H_2O$} are {-27, -45, -44, -62}, {+1, -17, -35, -16, -34}, and {+18, 0, -18, +1, -17}, respectively. The mass offset sets for the C-terminal x-ion and y-ion type sets can be calculated by shifting the masses of the c-ion and b-ion type sets by +27 and +18, respectively. According to the fragmentation method and the property of the data, all or some of the above ion types can be used flexibly.

The candidate sequences obtained in the step **120** are found by using the preprocessed data, which aim to provide more reliable information to generate the partial sequence. However, when insufficient data is provided by preprocessing, AACs are likely to be present in the candidate sequences. In the step **140,** if selected sequences with missing mass values exist, which means that the corresponding mass gaps are equal to the summation of at least two amino acids, the raw data may be used to find as many vertices as possible for the path in Stage 2. For the raw data, suppose that all ($m/z$) ratios have the opportunity to be created by singly, doubly or triply charged ions. Then the set with q mass/charge ratios $(m/z)_i$, i = 1,2, ..., q, can be converted to the mass set $m'_b$ of putative b-ion and the equivalent b-ion mass set $m'_y$ of putative y-ion, and each of the sets $m'_b$ and $m'_y$ has 3q elements. Although the number of mass values increases, only the range between the head mass and the tail mass of the AAC is considered and is relatively smaller when compared to that for the whole sequence. As shown in FIG. **4,** a gap being the mass summation of 4 amino acids is shown in Path 1. With more information provided by the raw data, the following cases can be found for the gap: (a) composition of amino acid and AAC, (b) composition of two AACs, (c) composition of tag and AAC, and (d) one tag. Note that a gap being the mass summation of more amino acids is effective in ensuring that valid paths can be formed. However, more candidate sequences may be generated and thus a longer time is required for peptide sequencing.

As shown in FIG. **3,** after finding the missing amino acids of AACs in step **141,** the sequences with the longest length of consecutive amino acids retrieved in AACs are selected as the candidate sequences (step **142**). If at least two candidate sequences remain after selection, a final decision is made based on the match error of the amino acids retrieved in AACs for each sequence (step **143).**

### E. Peptide Sequencing: Highest-Intensity-Tag based Sequencing Method

The mass/charge (m/z) ratio corresponding to the first or second highest intensity is first recognized to further infer the tag or the path. In the highest-intensity-tag based sequencing method, short tags with three amino acids are used in the tag-based methods, such as GutenTag, DirecTag, and NovoHCD. Although tags with shorter lengths can avoid introducing incorrect amino acids, the number of candidate tags is relatively larger and sometimes it is harder to infer the sequences due to insufficient information provided by the tag. Note that the length of the tag is not fixed and can be up to the length of the peptide if the data is complete, thus helping to reduce the search space. When a tag contains incorrect amino acids, usually it cannot be extended with valid prefix and suffix parts. In this case, the length of the tag is shortened by adaptively reducing the number of the higher-intensity data points used for the tag-finding algorithm. In addition, the vertex with the highest intensity may not definitely be present in the correct path due to uncertainty of the data. When valid paths cannot be found, it may be possible to infer the tag with the second highest intensity.

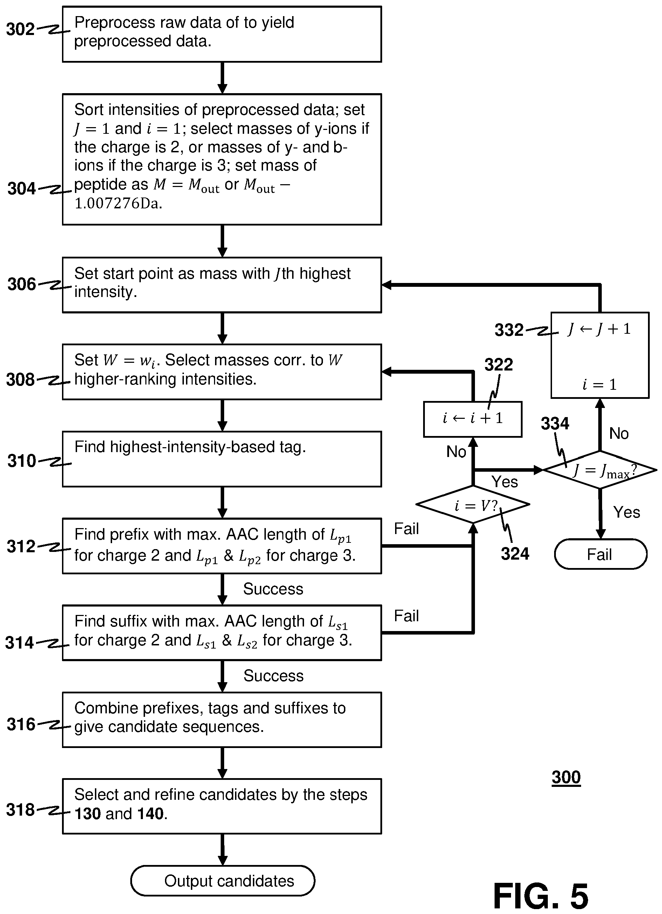

FIG. **5** depicts a flowchart showing exemplary steps used in a method **300** of highest-intensity-tag based sequencing. The method **300** commences at step **302** for preprocessing the raw data. The step **302** is the same as the step **110** of the two-stage sequencing method **100.** The method **300** then proceeds from the step **302** to steps **304, 306** and **308.**

In the steps **304, 306** and **308,** the intensities of the preprocessed data are sorted from the largest to the smallest with $J$ denoting the ranking of intensity. The mass/charge ratio with the highest intensity is then identified and the mass/charge ratio is converted to the corresponding mass of a b-ion. As a start, it is set as $J = 1$ and $i = 1$, and only the mass/charge ratio with a higher ranking $W = w_i$ ($w_1 > w_2 > \cdots > w_V$) is used in tag-finding processing.

In the steps **302** and **304,** for each sequence, a preprocessing unit also outputs (i) the selection of the mass set (i.e. set of y-ions, or set of y-ions and b-ions) based on the charge (most often 2 or 3), and (ii) the determination of the mass $M$ (= $M_{out}$ or $M_{out}$-1.007276 Da) of the whole sequence based on the isotopic pattern of the parent ion in a similar fashion as in the preprocessing step (i.e. the step **110**) as disclosed above, where $M_{out}$ is the mass output of the mass spectrometer corresponding to the highest intensity, which may be the monoisotopic peak or the peak that follows (with mass +1.007276 Da).

The selection of suitable mass set(s) for peptide sequencing is strongly based on the peptide design. In one embodiment, F is the head (fixed amino acid at the N-terminus), R or K is the tail (fixed amino acid at the C-terminus) and no P or other basic amino acids in the middle. Previous experimental data shows that the parent ion peak with charge 2 is the strongest peak while the one with charge 3 is very weak, and the y-ions are much stronger than the b-ions when the charge 2 peak were selected for MS/MS. In this case, only the MS/MS spectra from the charge 2 peak would be analysed. Furthermore, using the masses of y-ions would be sufficient to find all sequences and thus only y-ions are used. In another embodiment, H is the head, R is the tail and no P or other basic amino acids in the middle. In this case, the charge 3 peak may be much stronger than, or as strong as the charge 2 peak. The MS/MS spectra of one or both parent peaks would be analysed. When the charge is 2, using the masses of y-ions is sufficient to find all sequences and thus only y-ions are used. However, when the charge is 3, both y- and b-ions are used. In another embodiment, the head and tails are not basic amino acids, the charge is usually 2, and both y- and b-ions would be used.

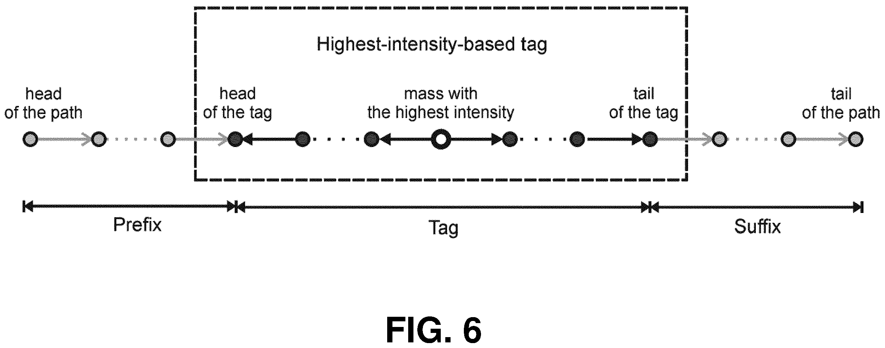

The method **300** then proceeds to step **310** to find the highest-intensity-based tag. Starting from the mass $m'_{B,J}$ of the b-ion or $m'_{Y,J}$ of the y-ion with the highest intensity, the highest-intensity-based tag is found by simultaneously connecting the vertices in the forward direction pointing to the tail vertex of the path and connecting the vertices in the backward direction pointing to the head vertex of the path. Here, the vertices have mass gaps being the mass $g_k$ ($k$ = 1, ..., $K$) of any amino acid and the length of the tag should be as long as possible (see FIG. **6**). The tags containing the amino acid with the highest intensity are obtained subsequently, and are called the highest-intensity-based tags. With knowledge of the masses of the head and the tail amino acids of a highest-intensity-based tag, the method **300** proceeds to step **312** to find the prefixes that can connect the head of the path to the head of the tags in the forward direction by using the method described in the step **120** of the two-stage sequencing method **100.** For the tags with valid prefixes, in step **314,** the suffix parts of the sequences can be further found by linking the tail of the tags to the tail of the path in the forward direction using a similar method.

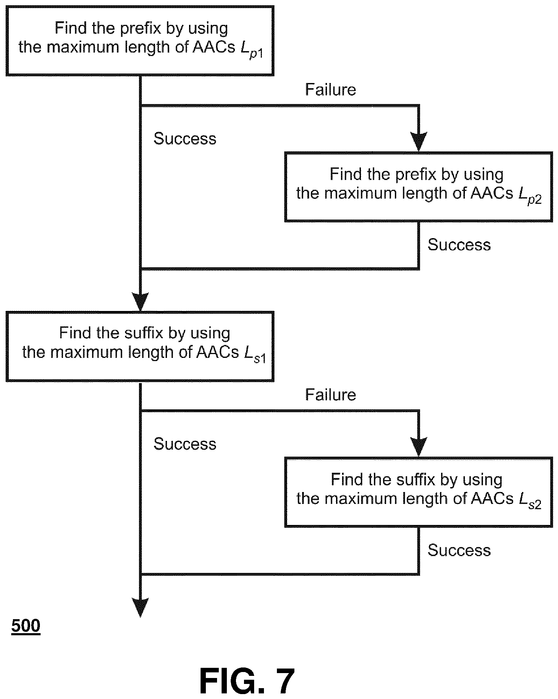

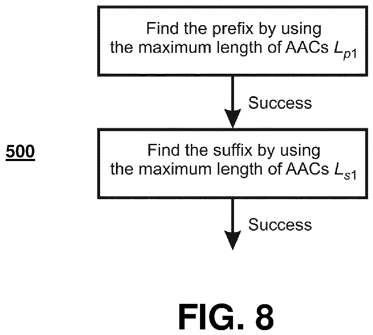

In the prefix and suffix finding process in the steps **312** and **314,** it is very important to set the maximum length of AACs properly such that more valid sequences can be found even when some of the masses are not reliable. The maximum length of AACs is set to limit a length of each AAC in the prefix or suffix. The preprocessed data are relatively reliable and provide a mass-intensity pattern for each output mass. Thus, we use the preprocessed data to first find a main path with one or more AACs. Moreover, the path connects the head amino acid for the prefix part, the tail amino acid for the suffix part, and the highest-intensity tag. If the maximum length of AACs is small (for example 3 or 4), then the searching time is short but some of the valid paths may not be found. In case the prefix part or the suffix part cannot be found, we can adjust the maximum length of AACs to a sufficiently large value (for example 10). Details of the prefix- and suffix-finding methods **500** for charge 3 and for charge 2 are illustrated in FIGS. **7** and **8,** respectively. Let the two maximum lengths of AACs for the prefix be $L_{p1}$ and $L_{p2}$ with $L_{p2} > L_{p1}$, and the two maximum lengths of AACs for the suffix be $L_{s1}$ and $L_{s2}$ with $L_{s2} > L_{s1}$. As shown in the AAC(b) shown in FIG. **4,** after the highest-intensity tag is obtained, we first use $L_{p1}$ to find the prefix. If a prefix cannot be found, we use $L_{p2}$ to find the prefix. After a valid prefix is found, we first use $L_{s1}$ to find the suffix. If it fails, we then use $L_{s2}$. Depending on the property of the spectra, the lengths in each one of the two pairs ($L_{p1}$, $L_{s1}$) and ($L_{p2}$, $L_{s2}$) can be equal or different. For the 4095-sequence data set, we use use $L_{p1} = L_{s1} = 3$ and $L_{s2} = 10 > L_{p2} = 6$. When the charge is 2, only $L_{p1}$ and $L_{s1}$ are considered, as shown in FIG. 7.

In step **316,** the candidate paths can be constructed by combining the three parts: prefix, tag, and suffix. In step **318,** one can follow the steps **130** and **140** of the two-stage sequencing method **100** to select and refine the sequences. Note that a larger value used for $W$ sometimes introduces one or more incorrect amino acids in the head and/or tail parts of a tag, while a smaller value used for $W$ may give a more reliable tag but the length of the tag may be limited. Therefore, in steps **322** and **324,** if no valid candidates can be found, one may attempt to reduce the value of W with W = $w_i$ by increasing $i$ by one, i.e. $i \leftarrow i + 1$, and repeat the tag-prefix-suffix finding procedure until the candidate sequence can be found or $i = V$ is reached (where $V$ is the maximum number of iterations).

In steps **332** and **334,** when the experimental mass with the highest intensity gives an unreliable message due to noise and uncertainty, a highest-intensity-based tag or a valid path with the highest-intensity-based tag is not found. In this case, the mass with the second highest intensity is used, by setting $J \leftarrow J + 1$ and $i = 1$, to find the second highest-intensity-based tag and the candidates. This process continues until the sequence is found or $J = J_{max}$ is reached (where $J_{max}$ is the maximum number of higher-ranking-intensity masses allowed to be the start point to find the tag).

In some embodiments, both y-ions and b-ions are considered in the sequencing as described above. To reduce the searching space and improve the accuracy for the sequencing, we consider the following three steps for the selection of the mass set: (1) selecting the mass ranges for both y-ions and b-ions; (2) eliminating the mass overlap between y-ions and b-ions; and (3) avoiding two masses representing the same peak of the spectrum coexisting in the path.

Step **1**. We use the masses of both y-ions and b-ions for sequencing. Depending on the properties of the spectra, we can select: (i) some of the masses of y-ions and some of the masses of b-ions; (ii) all masses of y-ions and some of the masses of b-ions; (iii) some of the masses of y-ions and all masses of b-ions; or (iv) all masses of y-ions and b-ions. When more masses of y-ions and/or b-ions are used, more noise and interference is introduced into the sequencing. Hence, we use as few mass values as possible with an aim to ensure that the valid sequences include the correct sequence. In other words, Case (i) as mentioned above is preferred.

In Case (i), the mass set $m'$ used to find the path is then given by $m' = \{m'_b, m'_y\} = \{$m'$_{b,1}, m'_{b,2}, ... , m'_{b,L}, m'_{y,1}, m'_{y,2}, ... , m'_{y,L}\}$. For the peptides in the 4095-sequence data set as described above, it has been observed that the second half part of the full path can be obtained by using the masses of y-ions, while the first half part is mainly found by using the masses of b-ions. If the mass set is $m' = \{m'_{b,1}, m'_{b,2}, ... , m'_{b,u}, m'_{y,u+1}, m'_{y,u+2}, ... , m'_{y,L}\}$, then the path does not contain both mass $m'_{b,i}$ and $m'_{y,i}$ for each i = 1,2, ..., L. One simple setting method is to set $u$ such that $m'_{b,u}$ is the largest value approaching M/2. Then the mass set m' used to find the path contains the masses of b-ions in $m'_b$ below M/2 and the masses of y-ions in $m'_y$ beyond or equal to $M/2$, i.e. $m'_{b,i} < M/2$ for i = 1,2, ... , $u$ and $m'_{y,i} \geq M/2$ for $i = u + 1, u + 2, ... , L$.

Step **2**. For some spectra, using only the masses in set $\{m'_{b,1}, m'_{b,2}, ... , m'_{b,u}\}$ with $m'_{b,i} < M/2$ for i = 1,2, ..., $u$ cannot fully cover the range of the first half of the path, and hence some information of y-ions is needed to obtain one or two correct masses located near $M/2$. Here we adjust the mass set for sequencing and include the masses of y-ions $m'_{y,i} \geq M/2 - e$ (here we set $e = 200$) for $i = v + 1, v + 2, ... , L$ and the masses of b-ions $m'_{b,i} < M/2$ for i = 1,2, ... , $u$, i.e. $m' = \{m'_{b,1}, m'_{b,2}, ... , m'_{b,u}, m'_{y,v}, m'_{y,v+1}, ... , m'_{y,u}, m'_{y,u+1}, ... , m'_{y,L}\}$ with $u > v$. The masses of the b-ions may overlap with the masses of the y-ions in the range from mass $M/2 - e$ to $M/2$, which implies that different peaks of the spectrum generate the same mass, i.e. $m'_{b,i} = m'_{y,i}$. We suppose that the mass pairs $P_1 = \{m'_{y,i}, m'_{y,i+1}\}$, $P_2 = \{m'_{y,i}, m'_{b,i+1}\}$, $P_3 = \{m'_{b,i}, m'_{y,i+1}\}$ and $P_4 = \{m'_{b,i}, m'_{b,i+1}\}$ are present in the spectrum when $m'_{b,i} = m'_{y,i}$ and $m_{b,i+1} = m'_{y,i+1}$. Then we merge the four pairs $P_1 \sim P_4$ into one pair. Generally, there is a deviation between the experimental and the theoretical masses. Then we give priority according to $P_1 > P_4 > P_3 > P_2$. When all four pairs are found, we select the mass pair $P_1$ and remove pairs $P_2$, $P_3$ and $P_4$. Similarly, if only the two mass pairs $P_2$ and $P_4$ (or $P_3$ and $P_4$) are found, we keep $P_4$ only.

Step **3**. According to the antisymmetric requirement [Sherenga], only mass $m'_{b,i}$ or $M - m'_{b,i}$ ($m'_{y,i}$ or $M - m'_{y,i}$) can be present in the path to avoid occurrence of the same peak two times in a spectrum. Suppose that mass $m'_{b,j}$ (or $m'_{y,j}$) is one of the masses for the tag. We remove mass $M - m'_{b,j}$ (or $M - m'_{v,j}$) in set m' to further find the prefix part. Next, suppose that mass $m'_{b,k}$ (or $m'_{y,k}$) is one of the masses for the prefix part. We remove mass $M - m'_{b,k}$ (or $M - m'_{y,k}$) in set m' and continue to find the suffix part.

In the above descriptions of the steps **304** and **306,** all the masses of a peptide and the corresponding calculations are assumed to be precise. In practice, the masses given by the spectra have a tolerance and hence all calculations are performed within a tolerance range.

_F. Checking Method Based on the Design of the Sequences_

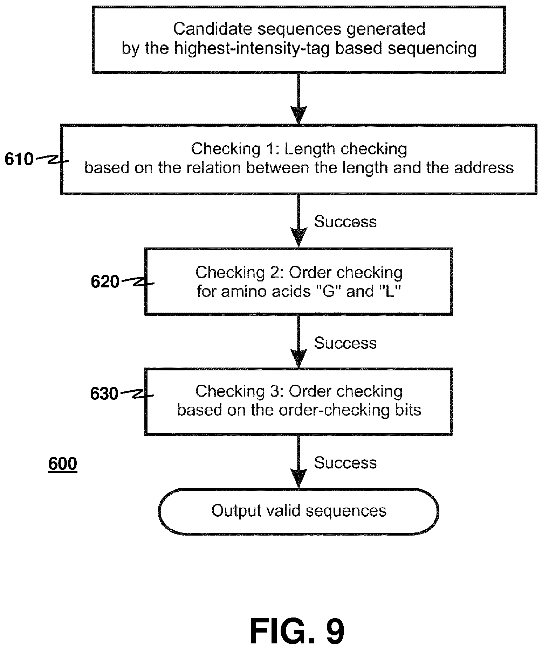

FIG. **9** shows an illustration of the checking method **600** for the candidate sequences. For each candidate sequence obtained by using the highest-intensity-based tag sequencing method **300,** we perform the following three checking steps based on the design of the peptides.

First, the length of a sequence can be 15, 16, 17, 18 or 19. As mentioned above in the design of the peptide sequences, the length of a sequence is directly related to its address in the whole block. Thus, the validation of a sequence can be determined by (i) evaluating an expected length of the sequence based on its address and (ii) comparing the expected length and the actual length. This checking is shown as checking step **610** in FIG. **9.**

Second, if there exists an output sequence with a valid length and the sequence contains the amino acid "L", the order of the amino acids "G" and "L" will be further checked. This checking is shown as checking step **620** in FIG. **9.** We first find the position $P_L$ of the last amino acid "L" and the position $P_G$ of the first amino acid "G", and then compare $P_L$ with $P_G$. If $P_L > P_G$, then the sequence is not valid and is to be discarded.

Third, if a sequence can be found successfully, we perform an order check based on the order-checking bits of the sequence. This checking is shown as checking step **630** in FIG. 9. According to the orders of the estimated symbol pairs $\{S_1, S_2\}$, $\{S_2, S_3\}$ and $\{S_{L'-1}, S_{L'}\}$, the order-checking bits are generated based on the pre-defined rules. The generated order-checking bits are compared against the corresponding bits in the estimated symbol $S_5$ to see if they are identical. As an example, if there is no error in the orders of $S_1$ and $S_2$, the generated order-checking bit for $\{S_1, S_2\}$ should match the first bit of the estimated symbol $S_5$. If the generated order-checking bits do not match all three bits of $S_5$ in the estimated sequence, the estimated sequence in the block should be removed.

Finally, if the sequence can pass all three checking processes, we output this sequence as a valid sequence.

Note that the three checking steps **610, 620, 630** may be performed in any order, not strictly in the above-mentioned order as shown in FIG. **9.** In addition, not all three checking steps **610, 620, 630** may be used together in verification. Any combination of one or more of the three checking steps **610, 620, 630** may be used.

Although the checking method **600** is illustrated for use in the highest-intensity-based tag sequencing method **300,** it is possible to use the checking method **600** for applications in the two-stage sequencing method **100.** Nonetheless, some of the checking steps **610, 620, 630** may be required to be adapted for the two-stage sequencing method **100.** For instance, it is possible that the requirement of a certain length may be observed even during the search for valid paths in the step **120.** The checking step **610** may not be necessary to be implemented in certain practical situations.

_G. Sample Data_

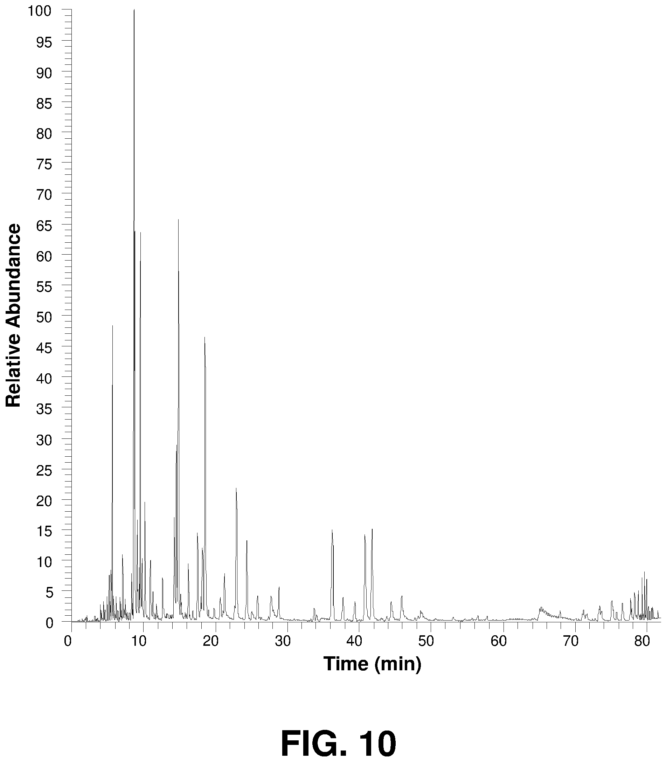

A peptide mixture encoded using the first 50 addresses in the 4095 peptides block as described above with F as head and R as tail (sequence details shown in Table **3**) was sequenced using the method described above. The TIC of the mixture is shown in FIG. **10.** All peptides were correctly sequenced.

Table **3**. Sequence details of the 50 peptides.

| ID | Sequence | Monoisotopic Mass |
|----|----------|-------------------|
| 1 | FGFVSEYYYYTVGSTAR (SEQ ID NO:1) | 2008.92071 |
| 2 | FFEYGGYYYTSYFGYEGR (SEQ ID NO:2) | 2267.94764 |
| 3 | FTFVYEYYYEYVYVYFEAR (SEQ ID NO:3) | 2613.17806 |
| 4 | FVAVLVYYYVSSETVLFGER (SEQ ID NO:4) | 2340.20423 |
| 5 | FYTFGYYYYAGFTFTTESYAR (SEQ ID NO:5) | 2617.14781 |
| 6 | FEYFESYYYSVVAGFFR (SEQ ID NO:6) | 2172.98332 |
| 7 | FVLVVEYYYGGGSTSTFR (SEQ ID NO:7) | 2043.99422 |
| 8 | FSFAAEYYYFFETYSFEFR (SEQ ID NO:8) | 2513.08924 |
| 9 | FTYEVSYYTYFYSTGSAGAR (SEQ ID NO:9) | 2332.03244 |
| 10 | FFTFTSYYTTASAGGTVEAER (SEQ ID NO:10) | 2305.0539 |
| 11 | FEFVTVYYTEAATAAYR (SEQ ID NO:11) | 2000.952 |
| 12 | FALVTVYYTVAYGASSER (SEQ ID NO:12) | 1995.99421 |
| 13 | FVTTYFYYTAEFSEASEYR (SEQ ID NO:13) | 2373.04776 |
| 14 | FYVGFTYYTSVAFFFEYFAR (SEQ ID NO:14) | 2524.17801 |
| 15 | FFYGTAYYTGVVFVFYAVYVR (SEQ ID NO:15) | 2531.2566 |
| 16 | FTTSTAYYTFYTFTVFR (SEQ ID NO:16) | 2114.99898 |
| 17 | FFYAESYYEYLEYVVFER (SEQ ID NO:17) | 2416.09399 |
| 18 | FGEAYSYYETSGFFVYGVR (SEQ ID NO:18) | 2241.0055 |
| 19 | FTAYTEYYEESSSSFAETGR (SEQ ID NO:19) | 2323.9757 |
| 20 | FFFAEFYYEVTFESEYEGGER (SEQ ID NO:20) | 2645.12746 |
| 21 | FSVETGYYEAYGEEYTR (SEQ ID NO:21) | 2062.87961 |
| 22 | FVFEFEYYESYYEYAVAR (SEQ ID NO:22) | 2374.04703 |
| 23 | FVVGASYYEGYYASYSSER (SEQ ID NO:23) | 2196.96402 |
| 24 | FGAFGSYYEFEGVVTESAVR (SEQ ID NO:24) | 2214.02696 |
| 25 | FYEVAYYYVYYSVVTSLAYER (SEQ ID NO:25) | 2647.25229 |
| 26 | FSTTSGYYVTFSYGEGR (SEQ ID NO:26) | 1920.85302 |
| 27 | FVAVAVYYVEFAVYYSYR (SEQ ID NO:27) | 2208.09321 |
| 28 | FYTAEYYYVVESGESFYTR (SEQ ID NO:28) | 2373.04776 |
| 29 | FEETAFYYVAFGASAYVSAR (SEQ ID NO:29) | 2248.04769 |
| 30 | FYGVEEYYVSAVTTYSGEYFR (SEQ ID NO:30) | 2529.13764 |
| 31 | FFEVFSYYVGATTSAYR (SEQ ID NO:31) | 2006.94145 |
| 32 | FETAFSYYVFFVEGFAVR (SEQ ID NO:32) | 2178.04626 |
| 33 | FSVASSYYAYESTVYVAVR (SEQ ID NO:33) | 2161.03681 |
| 34 | FGVEEFYYATVVTYGGSSER (SEQ ID NO:34) | 2260.03244 |
| 35 | FEEFYSYYAESSGTFGAAVVR (SEQ ID NO:35) | 2379.06955 |
| 36 | FGYGVSYYAVTTESETR (SEQ ID NO:36) | 1928.87923 |
| 37 | FEAAYEYYAAYTAGGEAR (SEQ ID NO:37) | 2001.87444 |
| 38 | FAFEYEYYASFAEVGTTVR (SEQ ID NO:38) | 2249.03171 |
| 39 | FEVFYTYYAGAAAEFGGFER (SEQ ID NO:39) | 2294.03203 |

(continued)

| ID | Sequence | Monoisotopic Mass |
|---|---|---|
| 40 | FLVATAYYAFVEYLVVVAESR (SEQ ID NO:40) | 2409.26207 |
| 41 | FEGVSEYYSYFSSVAGR (SEQ ID NO:41) | 1946.86867 |
| 42 | FVYTSSYYSTTFVAGEER (SEQ ID NO:42) | 2105.95822 |
| 43 | FSVTLFYYSELAFEVFFYR (SEQ ID NO:43) | 2427.18277 |
| 44 | FATVESYYSVGAFVEASSTR (SEQ ID NO:44) | 2170.02188 |
| 45 | FSAFYSYYSAEEEFSAYAVTR (SEQ ID NO:45) | 2487.09068 |
| 46 | FAVVVGYYSSETVYEAR (SEQ ID NO:46) | 1938.93636 |
| 47 | FTYAESYYSGSTGATETR (SEQ ID NO:47) | 1989.85921 |
| 48 | FTSAVEYYSFEGSVYESGR (SEQ ID NO:48) | 2176.95894 |
| 49 | FLEAAAYYLYVTFSVSAYVR (SEQ ID NO:49) | 2332.178 |
| 50 | FYGTAEYYGTEEFESGTYYER (SEQ ID NO:50) | 2561.05467 |

## H. Details of Embodiments of Present Disclosure

Embodiments of the present disclosure are elaborated as follows based on the details, examples, applications, etc., of the two-stage sequencing method **100,** the highest-intensity-tag based sequencing method **300** and the checking method **600** as disclosed above.

An aspect of the present disclosure is to provide a computer-implemented method for sequencing a data-encoded peptide from an experimental spectrum. The method provides one of the following three outputs. In the first output, which corresponds to the best case, an estimate of a peptide sequence is uniquely determined in sequencing the data-encoded peptide. In the second output, plural estimates of the peptide sequence are resulted. In the third output, no estimate is found.

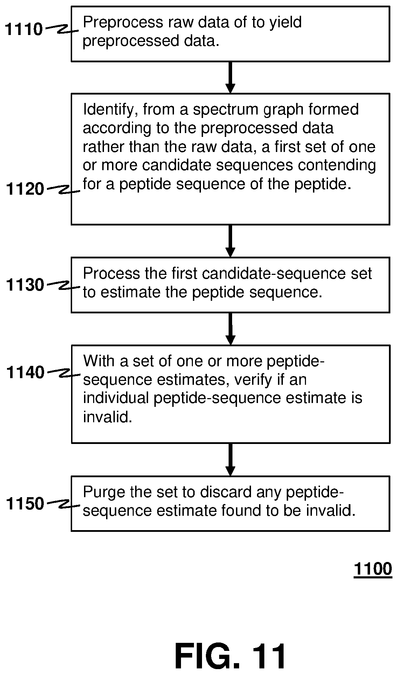

FIG. **11** depicts a flowchart showing exemplary steps of the disclosed computer-implemented method (referenced as **1100** for convenience). The method **1100** comprises steps **1110, 1120, 1130, 1140** and **1150.**

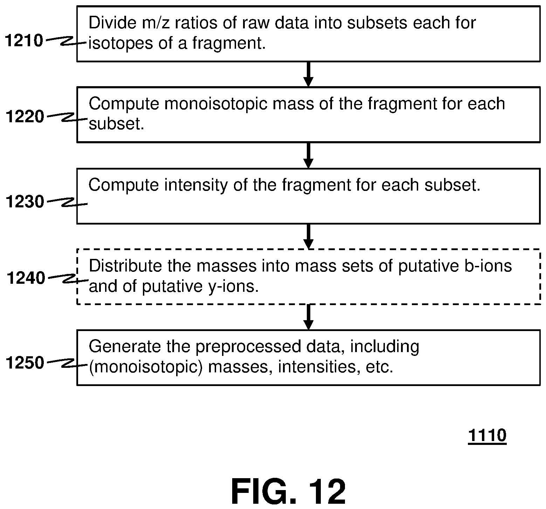

In the step **1110,** raw data of the experimental spectrum are preprocessed for removing uninterpretable peaks to yield preprocessed data. As mentioned above, the raw data of the experimental spectrum are data of a histogram of intensity versus mass/charge ratio as originally obtained in analyzing the data-encoded peptide by mass spectrometry. The step **1110** corresponds to the step **110** of the two-stage sequencing method **100** and the step **302** of the highest-intensity-tag based sequencing method **300.** The step **1110** is further elaborated with the aid of FIG. **12,** which depicts a flowchart showing exemplary steps executed in the step **1110.**

Exemplarily, the step **1110** comprises steps **1210, 1220, 1230** and **1250.** In the step **1210,** a set of mass/charge ratios of the raw data is divided into a plurality of subsets where each mass/charge ratio has a signal peak in the experimental spectrum. An individual subset consists of mass/charge ratios of isotopes of a fragment of the data-encoded peptide. In addition, the isotopes of the fragment have a same charge of positive integer value. Usually, the charge has a value of 1, 2 or 3. A monoisotopic mass of the fragment is then computed for the individual subset in the step **1220.** An intensity of the fragment is also computed in the step **1230** according to intensities associated with the mass/charge ratios in the individual subset. For instance, the intensity of the fragment may be computed by summing intensity values associated with different mass/charge ratios in the individual subset. The steps **1220** and **1230** are repeated for the plurality of subsets. In the step **1250,** the preprocessed data are generated. The preprocessed data includes a plurality of masses and a plurality of intensities. The plurality of masses is formed with the monoisotopic mass of the fragment for the individual subset. An individual mass in the plurality of masses is associated with a corresponding intensity in the plurality of intensities.

Preferably, the preprocessed data further includes a first mass set of putative b-ions and a second mass set of putative y-ions for the experimental spectrum. The step **1110** further comprises a step **1240** of distributing respective masses in the plurality of masses into the first and second mass sets before executing the step **1250.**

Refer to FIG. **11.** After the preprocessed data are obtained in the step **1110,** a first set of one or more candidate sequences contending for a peptide sequence of the peptide is identified from a spectrum graph in the step **1120,** where the spectrum graph is formed according to the preprocessed data rather than the raw data. As a result, a fewer number of candidate sequences is generated, thereby advantageously reducing a time cost in sequencing. The step **1120** corresponds to the step **120** of the two-stage sequencing method **100** and the steps **304, 306, 308, 310, 312, 314** and **316** of the highest-intensity-tag based sequencing method **300**.

After the first candidate-sequence set is obtained in the step **1120,** the first candidate-sequence set is processed in the step **1130** to estimate the peptide sequence.

After a set of one or more estimates of the peptide sequence is obtained, it is desirable to verify whether each estimate is valid such that any invalid estimate of the peptide sequence can be eliminated. The step **1140** is to verify whether an individual peptide-sequence estimate is invalid. In the step **1140,** whether the individual peptide-sequence estimate is invalid may be determined by: checking a length of the individual peptide-sequence estimate against a predetermined correct length of the peptide sequence; performing order checking for amino acids "G" and "L" in the individual peptide-sequence estimate; performing order checking based on order-checking bits carried in the data-encoded peptide; or any combination thereof. In the step **1150,** the set of one or more estimates of the peptide sequence is purged to remove any peptide-sequence estimate found to be invalid in the step **1140**.

Preferably, an individual candidate sequence in the first candidate-sequence set is identified in the step **1120** based on searching over appropriate mass set(s). If a parent ion peak identified in the experimental spectrum has a charge of value 2, preferably an individual candidate sequence in the first candidate-sequence set is identified or determined via searching over the second mass set. If the charge of the parent ion peak is of value 3, the individual candidate in the first candidate-sequence set is identified via searching over the first and second mass sets.

The step **1120** comprises the steps **130** and **140,** and is detailed as follows.

If the first candidate-sequence set consists of a single candidate sequence, the single candidate sequence in the first candidate-sequence set is assigned as one estimate of the peptide sequence. Otherwise, one or more candidate sequences are selected from the first candidate-sequence set to form a second set of one or more candidate sequences such that the one or more candidate sequences in the second candidate-sequence set are more probable to be the peptide sequence than non-selected candidate sequences in the first candidate-sequence set (step **130**).

If the second candidate-sequence set consists of a single candidate sequence, the single candidate sequence in the second candidate-sequence set is then assigned as one estimate of the peptide sequence.

If the second candidate-sequence set consists of plural candidate sequences that are free from any AAC, the plural candidate sequences in the second candidate-sequence set are then assigned as estimates of the peptide sequence.

If the second candidate-sequence set consists of plural candidate sequences that include one or more AACs, the raw data are then used to determine valid amino-acid sequences for the one or more AACs. After the valid amino-acid sequences are determined, the candidate sequences in the second candidate-sequence set are refined to generate a third set of one or more candidate sequences. The third candidate-sequence set is obtained from the second candidate-sequence set by using the determined valid amino-acid sequences to substitute the one or more AACs and then discarding any candidate sequence with an undetermined AAC (step **141**).

If the third candidate-sequence set consists of a single candidate sequence, the single candidate sequence in the third candidate-sequence set is then assigned as one estimate of the peptide sequence. Otherwise, one or more candidate sequences are selected from the third candidate-sequence set to form a fourth set of one or more candidate sequences such that the one or more candidate sequences in the fourth candidate-sequence set are more probable to be the peptide sequence than non-selected candidate sequences in the third candidate-sequence set (steps **142** and **143**).

If the fourth candidate-sequence set consists of a single candidate sequence, the single candidate sequence in the fourth candidate-sequence set is then assigned as one estimate of the peptide sequence. Otherwise, plural candidate sequences in the fourth candidate-sequence set are assigned as estimates of the peptide sequence.

In certain embodiments, the step **130** is set forth as follows. First, one or more candidate sequences with a longest length of consecutive amino acids among the candidate sequences in the first candidate-sequence set are selected to form a fifth candidate-sequence set (step **131**). If the fifth candidate-sequence set consists of a single candidate sequence, then the fifth candidate-sequence set is assigned to be the second candidate-sequence set. Otherwise, one or more candidate sequences with a largest number of amino acids among plural candidate sequences in the fifth candidate-sequence set are selected to form a sixth candidate-sequence set (step **132**). If the sixth candidate-sequence set consists of a single candidate sequence, then the sixth candidate-sequence set is assigned to be the second candidate-sequence set. Otherwise, one or more candidate sequences with a smallest match error among plural candidate sequences in the sixth candidate-sequence set are selected to form a seventh candidate-sequence set (step **133**). If the seventh candidate-sequence set consists of a single candidate sequence, then the seventh candidate-sequence set is assigned to be the second candidate-sequence set. Otherwise, one or more candidate sequences with a highest average intensity value of retrieved amino acids among plural candidate sequences in the seventh candidate-sequence set are selected to form an eighth candidate-sequence set (step **134**). If the eighth candidate-sequence set consists of a single candidate sequence, then the eighth candidate-sequence set is assigned to be the second candidate-sequence set. Otherwise, one or more candidate sequences with a highest number of occurrences for different ion types with different offsets among plural candidate sequences in the eighth candidate-sequence set are selected to form the second candidate-sequence set (step **135**).

In certain embodiments, the step **140** is set forth as follows. One or more candidate sequences from the third candidate-sequence set and with a largest number of amino acids among the determined valid amino-acid sequences are selected to form a ninth candidate-sequence set (step **142**). If the ninth candidate-sequence set consists of a single candidate sequence, then the ninth candidate-sequence set is assigned to be the fourth candidate-sequence set. Otherwise, one or more candidate sequences from the ninth candidate-sequence set and with a smallest match error among the determined valid amino-acid sequences are selected to form the fourth candidate-sequence set (step **143**).

The step **1120** may be realized as the step **120**. In certain embodiments, the step **1120** is set forth as follows.

One or more paths that are valid and that have a longest length in the spectrum graph are first identified, where a valid path begins at a head vertex, and ends at a tail vertex with a mass of the data-encoded peptide. Each of the identified one or more paths is then used to generate a new candidate sequence. The new candidate sequence is allocated to the first candidate-sequence set, unless the new candidate sequence is discarded due to some additional consideration.

The step **1120** may be realized as the steps **304, 306, 308, 310, 312, 314** and **316**. In certain embodiments, the step **1120** is set forth with the following steps.

<u>Step A.</u> Sort the plurality of masses in a descending order of corresponding intensities of respective masses to thereby form an ordered sequence of masses, where the ordered sequence of masses ranks the plurality of masses with a highest-ranking mass being associated with a highest corresponding intensity (step **304**).

<u>Step B.</u> Given a selected mass and a selected number of higher-ranking masses in the ordered sequence of masses, identify one or more highest-intensity-based tags in the spectrum graph (step **310**). An individual highest-intensity-based tag is a partial sequence consisting of a first amino acid having the selected mass and a plurality of remaining amino acids each having a mass selected from the selected number of higher-ranking masses.

<u>Step C.</u> Process the one or more highest-intensity-based tags. Processing the individual highest-intensity-based tag includes the following tasks: determining a prefix and a suffix for the individual highest-intensity-based tag (steps **312** and **314**); if the prefix and suffix are successfully determined, then combining the prefix, the individual highest-intensity-based tag and the suffix to form a new candidate sequence (step **316**); and allocating the new candidate sequence to the first candidate-sequence set.

In determining the prefix, the prefix is identified as a first path that is valid and that has a longest length in the spectrum graph, where the first path connects a head amino acid and a head of the individual highest-intensity-based tag, and one or more first AACs with a total length at most a first maximum length are allowable to be present in the identified first path. If the charge of the parent ion peak is of value 2, the first maximum length is set as $L_{p1}$ in determining the prefix. If the charge of the parent ion peak is of value 3, the first maximum length is initially set as $L_{p1}$ in finding the first path, and is relaxed to $L_{p2}$ with $L_{p2} > L_{p1}$ in case finding the first path under the first maximum length being $L_{p1}$ fails.

In determining the suffix, the suffix is identified as a second path that is valid and that has a longest length in the spectrum graph, where the second path connects a tail of the individual highest-intensity-based tag and a tail amino acid, and one or more second AACs with a total length at most a second maximum length are allowable to be present in the identified second path. If the charge of the parent ion peak is of value 2, the second maximum length is set as $L_{s1}$. If the charge of the parent ion peak is of value 3, the second maximum length is initially set as $L_{s1}$ in finding the second path, and is relaxed to $L_{s2}$ with $L_{s2} > L_{s1}$ if finding the second path under the second maximum length being $L_{s1}$ fails.

<u>Step D.</u> Sequentially use successive values in a strictly decreasing sequence of values as the selected number of higher-ranking masses to repeat Steps B and C until the first candidate-sequence set is not empty or the strictly decreasing sequence is exhausted (steps **322** and **324**).

<u>Step E.</u> Starting from the highest-ranking mass, sequentially use successive masses in the ordered sequence of masses as the selected mass to repeat Steps B-D until the first candidate-sequence set is not empty or a preselected number of higher-ranking masses in the ordered sequence of masses is reached (steps **332** and **334**). The preselected number is $J_{max}$.

The disclosed computer-implemented method is realizable by a computer system with appropriate programming. The computer system is implemented with one or more computers. An individual computer may be a general-purpose computer, a workstation, a computing server, a distributed server in a computing cloud, a notebook computer, a mobile computing device, etc.

**Claims**

1. A computer-implemented method for sequencing a data-encoded peptide from an experimental spectrum, raw data of the experimental spectrum being data of a histogram of intensity versus mass/charge ratio as originally obtained in analysing the data-encoded peptide by mass spectrometry, the method comprising:

   preprocessing the raw data for removing uninterpretable peaks to yield preprocessed data (110, 302, 1110);
   identifying, from a spectrum graph, a first candidate-sequence set (120, 1120) of one or more candidate sequences contending for a peptide sequence of the peptide,

wherein the spectrum graph is formed according to the preprocessed data rather than the raw data for generating a fewer number of candidate sequences to thereby reduce a time cost in sequencing;

processing the first candidate-sequence set to estimate the peptide sequence (130, 131, 132, 133, 134, 135, 1130, 140, 141, 142, 143, 1140);

after a set of one or more estimates of the peptide sequence is obtained, verifying whether an individual peptide-sequence estimate is invalid (610, 620, 630); and

purging the set of one or more estimates of the peptide sequence to remove any peptide-sequence estimate found to be invalid (1150),

wherein the preprocessing of the raw data (110, 302, 1110) to yield the preprocessed data comprises:

dividing a set of mass/charge ratios of the raw data into a plurality of subsets (1210) such that an individual subset consists of mass/charge ratios of isotopes of a fragment of the data-encoded peptide, an individual mass/charge ratio having a signal peak in the experimental spectrum, the isotopes of the fragment having a same charge of positive integer value;

computing a monoisotopic mass of the fragment for the individual subset (1220);

computing an intensity of the fragment (1230) according to the raw data and the mass/charge ratios in the individual subset; and

generating the preprocessed data (1250), wherein:

the preprocessed data includes a plurality of masses and a plurality of intensities,

wherein the plurality of masses is formed with the monoisotopic mass of the fragment for the individual subset, and wherein an individual mass is associated with a corresponding intensity; and

the preprocessed data further includes a first mass set of putative b-ions and a second mass set of putative y-ions for the experimental spectrum, wherein the first and second mass sets are generated by distributing respective masses in the plurality of masses into the first and second mass sets (1240);

and wherein the identifying of the first candidate-sequence set from the spectrum graph comprises the steps of:

(a) sorting the plurality of masses in a descending order of corresponding intensities of respective masses to thereby form an ordered sequence of masses, wherein the ordered sequence of masses ranks the plurality of masses with a highest-ranking mass being associated with a highest corresponding intensity;

(b) given a selected mass and a selected number of higher-ranking masses in the ordered sequence of masses, identifying one or more highest-intensity-based tags in the spectrum graph, wherein an individual highest-intensity-based tag is a partial sequence consisting of a first amino acid having the selected mass and a plurality of remaining amino acids each having a mass selected from the selected number of higher-ranking masses;

(c) processing the one or more highest-intensity-based tags, wherein processing the individual highest-intensity-based tag comprises:

determining a prefix and a suffix for the individual highest-intensity-based tag;

if the prefix and suffix are successfully determined, then combining the prefix, the individual highest-intensity-based tag and the suffix to form a new candidate sequence; and

allocating the new candidate sequence to the first candidate-sequence set;

(d) sequentially using successive values in a strictly decreasing sequence of values as the selected number of higher-ranking masses to repeat the steps (b) and (c) until the first candidate-sequence set is not empty or the strictly decreasing sequence is exhausted; and

(e) starting from the highest-ranking mass, sequentially using successive masses in the ordered sequence of masses as the selected mass to repeat the steps (b)-(d) until the first candidate-sequence set is not empty or a preselected number of higher-ranking masses in the ordered sequence of masses is reached;

**characterized in that** the determining of the prefix and suffix for the individual highest-intensity-based tag comprises:

identifying the prefix as a first path that is valid and that has a longest length in the spectrum graph, wherein:

the first path connects a head amino acid and a head of the individual highest-intensity-based tag;

one or more first amino acid combinations, AACs, with a total length at most a first maximum length are allowable to be present in the identified first path;

if a parent ion peak identified in the experimental spectrum has a charge of value 2, then the first maximum length is set as $L_{p1}$; and

if the charge of the parent ion peak is of value 3, then the first maximum length is initially set as $L_{p1}$ in finding the first path, and is relaxed to $L_{p2}$ in response to failure of finding the first path under the first maximum length being $L_{p1}$, where $L_{p2} > L_{p1}$; and

identifying the suffix as a second path that is valid and that has a longest length in the spectrum graph, wherein:

the second path connects a tail of the individual highest-intensity-based tag and a tail amino acid;

one or more second AACs with a total length at most a second maximum length are allowable to be present in the identified second path;

if the charge of the parent ion peak is of value 2, then the second maximum length is set as $L_{s1}$; and

if the charge of the parent ion peak is of value 3, then the second maximum length is initially set as $L_{s1}$ in finding the second path, and is relaxed to $L_{s2}$ in response to failure of finding the second path under the second maximum length being $L_{s1}$, where $L_{s2} > L_{s1}$.

2. The method of claim 1, wherein the identifying of the first candidate-sequence set (120, 1120) from the spectrum graph further comprises:

if a parent ion peak identified in the experimental spectrum has a charge of value 2, then identifying an individual candidate sequence in the first candidate-sequence set via searching over the second mass set; and

if the charge of the parent ion peak is of value 3, then identifying the individual candidate sequence in the first candidate-sequence set via searching over the first and second mass sets.

3. The method of claim 1, wherein the processing of the first candidate-sequence set to estimate the peptide sequence (130, 131, 132, 133, 134, 135, 1130, 140, 141, 142, 143, 1140) comprises:

if the first candidate-sequence set consists of a single candidate sequence, then assigning the single candidate sequence in the first candidate-sequence set as one estimate of the peptide sequence, else selecting one or more candidate sequences from the first candidate-sequence set to form a second candidate-sequence set of one or more candidate sequences such that the one or more candidate sequences in the second candidate-sequence set are more probable to be the peptide sequence than non-selected candidate sequences in the first candidate-sequence set;

if the second candidate-sequence set consists of a single candidate sequence, then assigning the single candidate sequence in the second candidate-sequence set as one estimate of the peptide sequence;

if the second candidate-sequence set consists of plural candidate sequences that are free from any amino acid combination (AAC), then assigning the plural candidate sequences in the second candidate-sequence set as estimates of the peptide sequence;

if the second candidate-sequence set consists of plural candidate sequences that include one or more AACs, then using the raw data to determine valid amino-acid sequences for the one or more AACs;

after the valid amino-acid sequences are determined, refining the candidate sequences in the second candidate-sequence set to generate a third candidate-sequence set of one or more candidate sequences, wherein the third candidate-sequence set is obtained from the second candidate-sequence set by using the determined valid amino-acid sequences to substitute the one or more AACs and then discarding any candidate sequence with an undetermined AAC;

if the third candidate-sequence set consists of a single candidate sequence, then assigning the single candidate sequence in the third candidate-sequence set as one estimate of the peptide sequence, else selecting one or more candidate sequences from the third candidate-sequence set to form a fourth candidate-sequence set of one or more candidate sequences such that the one or more candidate sequences in the fourth candidate-sequence set are more probable to be the peptide sequence than non-selected candidate sequences in the third candidate-sequence set; and

if the fourth candidate-sequence set consists of a single candidate sequence, then assigning the single candidate sequence in the fourth candidate-sequence set as one estimate of the peptide sequence, else assigning plural candidate sequences in the fourth candidate-sequence set as estimates of the peptide sequence.

4. The method of claim 3, wherein the selecting of the one or more candidate sequences from the first candidate-sequence set to form the second candidate-sequence set comprises:

selecting one or more candidate sequences with a longest length of consecutive amino acids among the candidate sequences in the first candidate-sequence set to form a fifth candidate-sequence set;

if the fifth candidate-sequence set consists of a single candidate sequence, then assigning the fifth candidate-sequence set to be the second candidate-sequence set, else selecting one or more candidate sequences with a largest number of amino acids among plural candidate sequences in the fifth candidate-sequence set to form a sixth candidate-sequence set;

if the sixth candidate-sequence set consists of a single candidate sequence, then assigning the sixth candidate-sequence set to be the second candidate-sequence set, else selecting one or more candidate sequences with a smallest match error among plural candidate sequences in the sixth candidate-sequence set to form a seventh candidate-sequence set;

if the seventh candidate-sequence set consists of a single candidate sequence, then assigning the seventh candidate-sequence set to be the second candidate-sequence set, else selecting one or more candidate sequences with a highest average intensity value of retrieved amino acids among plural candidate sequences in the seventh candidate-sequence set to form an eighth candidate-sequence set; and

if the eighth candidate-sequence set consists of a single candidate sequence, then assigning the eighth candidate-sequence set to be the second candidate-sequence set, else selecting one or more candidate sequences with a highest number of occurrences for different ion types with different offsets among plural candidate sequences in the eighth candidate-sequence set to form the second candidate-sequence set.

5. The method of claim 3, wherein the selecting of one or more candidate sequences from the third candidate-sequence set to form the fourth candidate-sequence comprises:

selecting, from the third candidate-sequence set, one or more candidate sequences with a largest number of amino acids among the determined valid amino-acid sequences to form a ninth candidate-sequence set; and

if the ninth candidate-sequence set consists of a single candidate sequence, then assigning the ninth candidate-sequence set to be the fourth candidate-sequence set, else selecting, from the ninth candidate-sequence set, one or more candidate sequences with a smallest match error among the determined valid amino-acid sequences to form the fourth candidate-sequence set.

6. The method of claim 1, wherein the verifying of whether the individual peptide-sequence estimate is invalid (610) comprises checking a length of the individual peptide-sequence estimate against a predetermined correct length of the peptide sequence.

7. The method of claim 1, wherein the verifying of whether the individual peptide-sequence estimate is invalid (620) comprises performing order checking for amino acids "G" and "L" in the individual peptide-sequence estimate.

8. The method of claim 1, wherein the verifying of whether the individual peptide-sequence estimate is invalid (630) comprises performing order checking based on order-checking bits carried in the data-encoded peptide.

**Patentansprüche**

1. Computer-implementiertes Verfahren zum Sequenzieren eines datenkodierten Peptids aus einem experimentellen Spektrum, wobei Rohdaten des experimentellen Spektrums Daten eines Histogramms der Intensität gegenüber dem Masse/Ladungs-Verhältnis sind, wie sie ursprünglich bei der Analyse des datenkodierten Peptids durch Massenspektrometrie erhalten wurden, wobei das Verfahren umfasst:

Vorverarbeiten der Rohdaten zum Entfernen nicht interpretierbarer Peaks, um vorverarbeitete Daten (110, 302, 1110) zu erhalten;

Identifizieren eines ersten Kandidatensequenz-Satzes (120, 1120) aus einer oder mehreren Kandidatensequenzen, die für eine Peptidsequenz des Peptids in Frage kommen, aus einem Spektraldiagramm, wobei das Spektraldiagramm gemäß den vorverarbeiteten Daten und nicht gemäß den Rohdaten gebildet wird, um eine geringere Anzahl von Kandidatensequenzen zu erzeugen und dadurch die Zeitkosten bei der Sequenzierung zu reduzieren;

Verarbeiten des ersten Kandidatensequenzsatzes, um die Peptidsequenz zu schätzen (130, 131, 132, 133, 134, 135, 1130, 140, 141, 142, 143, 1140);

nachdem ein Satz von einer oder mehreren Schätzungen der Peptidsequenz erhalten wurde, Überprüfen, ob eine einzelne Peptidsequenzschätzung ungültig ist (610, 620, 630); und

Bereinigen des Satzes von einer oder mehreren Schätzungen der Peptidsequenz, um jede Peptidsequenzschätzung zu entfernen, die als ungültig befunden wurde (1150),

wobei die Vorverarbeitung der Rohdaten (110, 302, 1110), um die vorverarbeiteten Daten zu erhalten, umfasst:

Aufteilen eines Satzes von Masse/Ladungs-Verhältnissen der Rohdaten in eine Vielzahl von Teilmengen (1210), so dass eine einzelne Teilmenge aus Masse/Ladungs-Verhältnissen von Isotopen eines Fragments des datencodierten Peptids besteht, wobei ein einzelnes Masse/Ladungs-Verhältnis einen Signalpeak im experimentellen Spektrum aufweist, wobei die Isotope des Fragments eine gleiche Ladung mit einem positiven ganzzahligen Wert aufweisen;

Berechnen einer monoisotopischen Masse des Fragments für die individuelle Teilmenge (1220);

Berechnen einer Intensität des Fragments (1230) gemäß den Rohdaten und den Masse/Ladungs-Verhältnissen in der individuellen Teilmenge; und

Erzeugen der vorverarbeiteten Daten (1250), wobei:

die vorverarbeiteten Daten eine Vielzahl von Massen und eine Vielzahl von Intensitäten umfassen, wobei die Vielzahl von Massen mit der monoisotopischen Masse des Fragments für die individuelle Teilmenge gebildet wird, und wobei eine individuelle Masse mit einer entsprechenden Intensität assoziiert ist; und

die vorverarbeiteten Daten ferner einen ersten Massensatz von mutmaßlichen b-Ionen und einen zweiten Massensatz von mutmaßlichen y-Ionen für das experimentelle Spektrum umfassen, wobei die ersten und zweiten Massensätze durch Verteilen jeweiliger Massen in der Vielzahl von Massen in die ersten und zweiten Massensätze (1240) erzeugt werden;

und wobei das Identifizieren des ersten Kandidatensequenzsatzes aus dem Spektraldiagramm die folgenden Schritte umfasst:

(a) Sortieren der Vielzahl von Massen in einer absteigenden Reihenfolge der entsprechenden Intensitäten der jeweiligen Massen, um dadurch eine geordnete Sequenz von Massen zu bilden, wobei die geordnete Sequenz von Massen die Vielzahl von Massen mit einer höchstrangigen Masse, die mit einer höchsten entsprechenden Intensität verbunden ist, reiht;

(b) Identifizieren einer oder mehrerer auf der höchsten Intensität basierender Markierungen in dem Spektraldiagramm bei einer gegebenen ausgewählten Masse und einer ausgewählten Anzahl von höherrangigen Massen in der geordneten Sequenz von Massen, wobei eine einzelne auf der höchsten Intensität basierende Markierung eine Teilsequenz ist, die aus einer ersten Aminosäure mit der ausgewählten Masse und einer Vielzahl von verbleibenden Aminosäuren besteht, die jeweils eine aus der ausgewählten Anzahl von höherrangigen Massen ausgewählte Masse aufweisen;

(c) Verarbeiten der einen oder mehreren auf der höchsten Intensität basierenden Markierungen, wobei das Verarbeiten der einzelnen auf der höchsten Intensität basierenden Markierung umfasst:

Bestimmen eines Präfixes und eines Suffixes für die individuelle Markierung auf Basis der höchsten Intensität;

wenn das Präfix und das Suffix erfolgreich bestimmt sind, dann Kombinieren des Präfixes, des individuellen Kennzeichens auf Basis der höchsten Intensität und des Suffixes, um eine neue Kandidatensequenz zu bilden; und

Zuordnen der neuen Kandidatensequenz zu dem ersten Kandidatensequenzsatz;

(d) sequentielles Verwenden aufeinanderfolgender Werte in einer streng absteigenden Folge von Werten als die ausgewählte Anzahl von höherrangigen Massen, um die Schritte (b) und (c) zu wiederholen, bis der erste Kandidatensequenzsatz nicht leer ist oder die streng absteigende Folge erschöpft ist; und

(e) ausgehend von der höchstrangigen Masse werden aufeinanderfolgende Massen in der geordneten Folge von Massen als die ausgewählte Masse verwendet, um die Schritte (b)-(d) zu wiederholen, bis die erste Kandidatensequenzsatz nicht leer ist oder eine vorgewählte Anzahl von höherrangigen Massen in der geordneten Folge von Massen erreicht ist;

**dadurch gekennzeichnet, dass** das Bestimmen des Präfixes und des Suffixes für das individuelle, auf der höchsten Intensität basierende Markierung umfasst:

Identifizieren des Präfixes als einen ersten Pfad, der gültig ist und der eine längste Länge in dem Spektraldiagramm hat, wobei:

der erste Pfad eine Kopf-Aminosäure und einen Kopf der individuellen Markierung auf Basis der höchsten Intensität verbindet;

eine oder mehrere erste Aminosäurekombinationen, AACs, mit einer Gesamtlänge von höchstens einer ersten Maximallänge in dem identifizierten ersten Pfad vorhanden sein dürfen;

wenn ein im experimentellen Spektrum identifizierter Eltern-Ionen-Peak eine Ladung des Wertes 2 hat, wird die erste maximale Länge als $L_{p1}$ festgelegt; und

wenn die Ladung des Eltern-Ionen-Peaks den Wert 3 hat, dann wird die erste maximale Länge anfänglich als $L_{p1}$ bei der Suche nach dem ersten Pfad eingestellt und auf $L_{p2}$ entspannt als Reaktion auf das Scheitern der Suche nach dem ersten Pfad unter der ersten maximalen Länge, die ist, wobei $L_{p2}>L_{p1}$;

und

Identifizieren des Suffixes als einen zweiten Pfad, der gültig ist und der eine längste Länge in dem Spektraldiagramm hat, wobei:

der zweite Pfad ein Ende der individuellen, auf höchster Intensität basierenden Markierung und eine End-Aminosäure verbindet;

eine oder mehrere zweite AACs mit einer Gesamtlänge von höchstens einer zweiten Maximallänge in dem identifizierten zweiten Pfad vorhanden sein dürfen;

wenn die Ladung des Eltern-Ionen-Peaks den Wert 2 hat, dann wird die zweite maximale Länge als $L_{s1}$ festgelegt; und

wenn die Ladung des Eltern-Ionen-Peaks den Wert 3 hat, dann wird die zweite maximale Länge bei der Suche nach dem zweiten Pfad anfänglich auf $L_{s1}$ gesetzt und auf $L_{s2}$ entspannt als Reaktion auf das Scheitern der Suche nach dem zweiten Pfad unter der zweiten maximalen Länge, die $L_{s1}$ ist, wobei $L_{s2}>L_{s1}$.

2. Verfahren nach Anspruch 1, wobei das Identifizieren des ersten Kandidatensequenz-Satzes (120, 1120) aus dem Spektraldiagramm ferner umfasst:

wenn ein im experimentellen Spektrum identifizierter Eltern-Ionen-Peak eine Ladung des Wertes 2 hat, dann Identifizieren einer individuellen Kandidatensequenz in dem ersten Kandidatensequenz-Satz durch Suchen über den zweite Massen-Satz; und

wenn die Ladung des Eltern-Ionen-Peaks den Wert 3 hat, dann Identifizieren der individuellen Kandidatensequenz in dem ersten Kandidatensequenz-Satz durch Suchen über den erste und zweite Massen-Satz.

3. Verfahren nach Anspruch 1, wobei die Verarbeitung des ersten Kandidatensequenz-Satzes zur Schätzung der Peptidsequenz (130, 131, 132, 133, 134, 135, 1130, 140, 141, 142, 143, 1140) umfasst:

wenn der erste Kandidatensequenz-Satz aus einer einzigen Kandidatensequenz besteht, dann Zuordnen der einzigen Kandidatensequenz in dem ersten Kandidatensequenz-Satz als eine Schätzung der Peptidsequenz, andernfalls Auswählen einer oder mehrerer Kandidatensequenzen aus dem ersten Kandidatensequenz-Satz, um einen zweiten Kandidatensequenz-Satz aus einer oder mehreren Kandidatensequenzen zu bilden, so dass die eine oder mehreren Kandidatensequenzen in dem zweiten Kandidatensequenz-Satz wahrscheinlicher die Peptidsequenz sind als nicht ausgewählte Kandidatensequenzen in dem ersten Kandidatensequenz-Satz;

wenn der zweite Kandidatensequenz-Satz aus einer einzigen Kandidatensequenz besteht, dann Zuordnen der einzigen Kandidatensequenz in dem zweiten Kandidatensequenz-Satz als eine Schätzung der Peptidsequenz;

wenn der zweite Kandidatensequenz-Satz aus mehreren Kandidatensequenzen besteht, die frei von jeder Aminosäurekombination (AAC) sind, dann Zuordnung der mehreren Kandidatensequenzen im zweiten Kandidatensequenz-Satz als Schätzungen der Peptidsequenz;

wenn der zweite Kandidatensequenz-Satz aus mehreren Kandidatensequenzen besteht, die eine oder mehrere AACs enthalten, dann Verwendung der Rohdaten, um gültige Aminosäuresequenzen für die eine oder mehrere AACs zu bestimmen;

nachdem die gültigen Aminosäuresequenzen bestimmt sind, Verfeinern der Kandidatensequenzen in dem zweiten Kandidatensequenz-Satz, um einen dritten Kandidatensequenz-Satz von einer oder mehreren Kandidatensequenzen zu erzeugen, wobei der dritte Kandidatensequenz-Satz aus dem zweiten Kandidatensequenz-Satz erhalten wird, indem die bestimmten gültigen Aminosäuresequenzen verwendet werden, um die eine oder mehreren AACs zu ersetzen, und dann jede Kandidatensequenz mit einer unbestimmten AAC verworfen wird;

wenn der dritte Kandidatensequenz-Satz aus einer einzigen Kandidatensequenz besteht, dann Zuordnen der einzigen Kandidatensequenz in dem dritten Kandidatensequenz-Satz als eine Schätzung der Peptidsequenz, andernfalls Auswählen einer oder mehrerer Kandidatensequenzen aus dem dritten Kandidatensequenz-Satz, um einen vierten Kandidatensequenz-Satz aus einer oder mehreren Kandidatensequenzen zu bilden, so dass die eine oder mehreren Kandidatensequenzen in dem vierten Kandidatensequenz-Satz wahrscheinlicher die Peptidsequenz sind als nicht ausgewählte Kandidatensequenzen in dem dritten Kandidatensequenz-Satz; und

wenn der vierte Kandidatensequenz-Satz aus einer einzelnen Kandidatensequenz besteht, dann Zuordnen der einzelnen Kandidatensequenz in dem vierten Kandidatensequenz-Satz als eine Schätzung der Peptidsequenz, ansonsten Zuordnen mehrerer Kandidatensequenzen in dem vierten Kandidatensequenz-Satz als Schätzungen der Peptidsequenz.

4. Verfahren nach Anspruch 3, wobei das Auswählen der einen oder mehreren Kandidatensequenzen aus dem ersten Kandidatensequenz-Satz zur Bildung des zweiten Kandidatensequenz-Satzes umfasst:

Auswählen einer oder mehrerer Kandidatensequenzen mit einer längsten Länge von aufeinanderfolgenden Aminosäuren unter den Kandidatensequenzen im ersten Kandidatensequenzsatz, um einen fünften Kandidatensequenzsatz zu bilden;

wenn der fünfte Kandidatensequenz-Satz aus einer einzigen Kandidatensequenz besteht, wird der fünfte Kandidatensequenz-Satz dem zweiten Kandidatensequenz-Satz zugeordnet, andernfalls werden eine oder mehrere Kandidatensequenzen mit einer größten Anzahl von Aminosäuren unter mehreren Kandidatensequenzen im fünften Kandidatensequenz-Satz ausgewählt, um einen sechsten Kandidatensequenz-Satz zu bilden;

wenn der sechste Kandidatensequenz-Satz aus einer einzigen Kandidatensequenz besteht, dann Zuweisung des sechsten Kandidatensequenz-Satzes zum zweiten Kandidatensequenz-Satz, andernfalls Auswahl einer oder mehrerer Kandidatensequenzen mit einem kleinsten Übereinstimmungsfehler unter mehreren Kandidatensequenzen im sechsten Kandidatensequenz-Satz, um einen siebten Kandidatensequenz-Satz zu bilden;

wenn der siebte Kandidatensequenz-Satz aus einer einzigen Kandidatensequenz besteht, Zuordnen des siebten Kandidatensequenz-Satzes zum zweiten Kandidatensequenz-Satz, andernfalls Auswählen einer oder mehrerer Kandidatensequenzen mit einem höchsten durchschnittlichen Intensitätswert der abgerufenen Aminosäuren unter mehreren Kandidatensequenzen im siebten Kandidatensequenz-Satz, um einen achten Kandidatensequenz-Satz zu bilden; und

wenn der achte Kandidatensequenz-Satz aus einer einzigen Kandidatensequenz besteht, Zuordnen des achten Kandidatensequenz-Satzes zum zweiten Kandidatensequenz-Satz, andernfalls Auswählen einer oder mehrerer Kandidatensequenzen mit einer höchsten Anzahl des Auftretens für verschiedene Ionenarten mit verschiedenen Offsets unter mehreren Kandidatensequenzen in dem achten Kandidatensequenz-Satz, um den zweiten Kandidatensequenz-Satz zu bilden.

5. Verfahren nach Anspruch 3, wobei die Auswahl einer oder mehrerer Kandidatensequenzen aus dem dritten Kandidatensequenz-Satz zur Bildung der vierten Kandidatensequenz umfasst:

Auswählen einer oder mehrerer Kandidatensequenzen mit der größten Anzahl von Aminosäuren unter den ermittelten gültigen Aminosäuresequenzen aus dem dritten Kandidatensequenz-Satz, um einen neunten Kandidatensequenz-Satz zu bilden; und

wenn der neunte Kandidatensequenzsatz aus einer einzigen Kandidatensequenz besteht, Zuordnen des neunten Kandidatensequenz-Satzes als viertes Kandidatensequenz-Satz, andernfalls Auswählen einer oder mehrerer Kandidatensequenzen mit einem kleinsten Übereinstimmungsfehler unter den ermittelten gültigen Aminosäuresequenzen aus dem neunten Kandidatensequenzsatz, um den vierten Kandidatensequenzsatz zu bilden.

**6.** Verfahren nach Anspruch 1, wobei das Überprüfen, ob die individuelle Peptidsequenz-Schätzung ungültig ist (610), das Überprüfen einer Länge der individuellen Peptidsequenz-Schätzung gegenüber einer vorbestimmten korrekten Länge der Peptidsequenz umfasst.

**7.** Verfahren nach Anspruch 1, wobei die Überprüfung, ob die individuelle Peptidsequenz-Schätzung ungültig ist (620), die Durchführung einer Ordnungsprüfung für die Aminosäuren "G" und "L" in der individuellen Peptidsequenz-Schätzung umfasst.

**8.** Verfahren nach Anspruch 1, wobei die Überprüfung, ob die individuelle Peptidsequenz-Schätzung ungültig ist (630), die Durchführung einer Ordnungsprüfung auf der Grundlage von Ordnungsprüfungsbits umfasst, die in dem datencodierten Peptid enthalten sind.


**Revendications**

**1.** Procédé implémenté par ordinateur pour séquencer un peptide codé par des données à partir d'un spectre expérimental, les données brutes du spectre expérimental étant des données d'un histogramme de l'intensité vs le rapport masse/charge tel qu'obtenu à l'origine lors de l'analyse du peptide codé par des données par spectrométrie de masse, le procédé comprenant :

le prétraitement des données brutes pour éliminer les pics ininterprétables afin d'obtenir des données prétraitées (110, 302, 1110) ;
l'identification, à partir d'un graphe spectral, d'un premier ensemble de séquences candidates (120, 1120) d'une ou de plusieurs séquences candidates se disputant une séquence peptidique du peptide, le graphe spectral étant formé en fonction des données prétraitées plutôt que des données brutes pour générer un nombre inférieur de séquences candidates, pour ainsi réduire le coût du séquençage ;
le traitement du premier ensemble de séquences candidates pour estimer la séquence peptidique (130, 131, 132, 133, 134, 135, 1130, 140, 141, 142, 143, 1140) ;
après obtention d'un ensemble d'une ou de plusieurs estimations de la séquence peptidique, la vérification si une estimation de séquence peptidique individuelle est invalide (610, 620, 630) ; et
la purge de l'ensemble d'une ou de plusieurs estimations de la séquence peptidique pour supprimer toute estimation de séquence peptidique jugée invalide (1150),
dans lequel le prétraitement des données brutes (110, 302, 1110) pour produire les données prétraitées comprend :

la division d'un ensemble de rapports masse/charge des données brutes en une pluralité de sous-ensembles (1210) de sorte qu'un sous-ensemble individuel soit constitué de rapports masse/charge d'isotopes d'un fragment du peptide codé par des données, un rapport masse/charge individuel ayant un pic de signal dans le spectre expérimental, les isotopes du fragment ayant une même charge de valeur entière positive ;
le calcul d'une masse monoisotopique du fragment pour le sous-ensemble individuel (1220) ;
le calcul d'une intensité du fragment (1230) en fonction des données brutes et des rapports masse/charge dans le sous-ensemble individuel ; et
la génération des données prétraitées (1250), dans lequel :

les données prétraitées comprennent une pluralité de masses et une pluralité d'intensités, la pluralité de masses étant formée avec la masse monoisotopique du fragment pour le sous-ensemble individuel, et une masse individuelle étant associée à une intensité correspondante ; et
les données prétraitées comprennent en outre un premier ensemble de masse d'ions b putatifs et un second ensemble de masse d'ions y putatifs pour le spectre expérimental, les premier et second ensembles de masse étant générés par distribution des masses respectives dans la pluralité de masses dans les premier et second ensembles de masse (1240) ;
et dans lequel l'identification du premier ensemble de séquences candidates à partir du graphe spectral comprend les étapes de :

(a) tri de la pluralité de masses dans un ordre décroissant d'intensités correspondantes de masses respectives pour former ainsi une séquence ordonnée de masses, la séquence ordonnée de masses classant la pluralité de masses avec une masse de rang le plus élevé qui est associée à une intensité correspondante la plus élevée ;

(b) étant donné une masse sélectionnée et un nombre sélectionné de masses de rang supérieur dans la séquence ordonnée de masses, identification d'une ou plusieurs étiquettes basées sur la plus haute intensité dans le graphe spectral, une étiquette basée sur la plus haute intensité individuelle étant une séquence partielle constituée d'un premier acide aminé ayant la masse sélectionnée et d'une pluralité d'acides aminés restants ayant chacun une masse sélectionnée parmi le nombre sélectionné de masses de rang supérieur;

(c) traitement de la ou des étiquettes basées sur la plus haute intensité, le traitement de l'étiquette basée sur la plus haute intensité individuelle comprenant :

la détermination d'un préfixe et d'un suffixe pour l'étiquette basée sur la plus haute intensité individuelle ;
si le préfixe et le suffixe sont déterminés avec succès, alors la combinaison du préfixe, de l'étiquette basée sur la plus haute intensité individuelle et du suffixe pour former une nouvelle séquence candidate ; et
l'attribution de la nouvelle séquence candidate au premier ensemble de séquences candidates ;

(d) l'utilisation de manière séquentielle de valeurs successives dans une séquence strictement décroissante de valeurs comme le nombre sélectionné de masses de rang supérieur pour répéter les étapes (b) et (c) jusqu'à ce que le premier ensemble de séquences candidates ne soit pas vide ou que la séquence strictement décroissante soit épuisée ; et

(e) en partant de la masse de rang le plus élevé, l'utilisation de manière séquentielle de masses successives dans la séquence ordonnée de masses comme la masse sélectionnée pour répéter les étapes (b) à (d) jusqu'à ce que le premier ensemble de séquences candidates ne soit pas vide ou qu'un nombre présélectionné de masses de rang supérieur dans la séquence ordonnée de masses soit atteint ;

**caractérisé en ce que** la détermination du préfixe et du suffixe pour l'étiquette basée sur la plus haute intensité individuelle comprend :

l'identification du préfixe comme premier chemin valide et qui a la longueur la plus longue dans le graphe spectral, dans lequel :

le premier chemin relie un acide aminé de tête et une tête de l'étiquette basée sur la plus haute intensité individuelle ;
une ou plusieurs premières combinaisons d'acides aminés, AAC, d'une longueur totale ne dépassant pas une première longueur maximale, peuvent être présentes dans le premier chemin identifié ;
si un pic ionique parent identifié dans le spectre expérimental a une charge de valeur 2, alors la première longueur maximale est fixée à $L_{p1}$ ; et
si la charge du pic ionique parent est de valeur 3, alors la première longueur maximale est initialement fixée à $L_{p1}$ pour trouver le premier chemin, et est relâchée jusqu'à $L_{p2}$ en réponse à l'échec de la recherche du premier chemin sous la première longueur maximale étant $L_{p1}$, où $L_{p2} > L_{p1}$ ;
et

l'identification du suffixe comme un second chemin valide et ayant une longueur la plus grande dans le graphe spectral, dans lequel :

le deuxième chemin relie une queue de l'étiquette basée sur la plus haute intensité individuelle et un acide aminé de queue ;
un ou plusieurs deuxièmes AAC dotés d'une longueur totale ne dépassant pas une deuxième longueur maximale peuvent être présents dans le deuxième chemin identifié ;
si la charge du pic ionique parent est de valeur 2, alors la deuxième longueur maximale est fixée à $L_{s1}$ ; et
si la charge du pic ionique parent est de valeur 3, alors la deuxième longueur maximale est initialement fixée à $L_{s1}$ pour trouver le deuxième chemin, et est relâchée jusqu'à $L_{s2}$ en réponse à l'échec de la recherche du second chemin sous la seconde longueur maximale étant $L_{s1}$, où $L_{s2} > L_{s1}$.

**2.** Procédé selon la revendication 1, dans lequel l'identification du premier ensemble de séquences candidates (120, 1120) à partir du graphe spectral comprend en outre :

si un pic ionique parent identifié dans le spectre expérimental a une charge de valeur 2, l'identification d'une séquence candidate individuelle dans le premier ensemble de séquences candidates par recherche dans le second ensemble de masses ; et
si la charge du pic ionique parent est de valeur 3, l'identification de la séquence candidate individuelle dans le premier ensemble de séquences candidates par recherche dans les premier et second ensembles de masse.

**3.** Procédé selon la revendication 1, dans lequel le traitement du premier ensemble de séquences candidates pour estimer la séquence peptidique (130, 131, 132, 133, 134, 135, 1130, 140, 141, 142, 143, 1140) comprend :

si le premier ensemble de séquences candidates est constitué d'une seule séquence candidate, alors l'attribution de la séquence candidate unique dans le premier ensemble de séquences candidates comme une estimation de la séquence peptidique, sinon la sélection d'une ou plusieurs séquences candidates dans le premier ensemble de séquences candidates pour former un second ensemble de séquences candidates d'une ou plusieurs séquences candidates de sorte que la ou les séquences candidates dans le second ensemble de séquences candidates soient plus susceptibles d'être la séquence peptidique que les séquences candidates non sélectionnées dans le premier ensemble de séquences candidates ;
si le deuxième ensemble de séquences candidates est constitué d'une seule séquence candidate, l'attribution de la séquence candidate unique dans le deuxième ensemble de séquences candidates comme une estimation de la séquence peptidique ;
si le second ensemble de séquences candidates consiste en plusieurs séquences candidates exemptes de toute combinaison d'acides aminés (AAC), alors l'attribution des séquences candidates du second ensemble de séquences candidates comme estimations de la séquence peptidique ;
si le deuxième ensemble de séquences candidates consiste en plusieurs séquences candidates qui comprennent un ou plusieurs AAC, alors l'utilisation des données brutes pour déterminer les séquences d'acides aminés valides pour un ou plusieurs AAC ;
après détermination des séquences d'acides aminés valides, le raffinage des séquences candidates dans le deuxième ensemble de séquences candidates pour générer un troisième ensemble de séquences candidates d'une ou de plusieurs séquences candidates, le troisième ensemble de séquences candidates étant obtenu à partir du deuxième ensemble de séquences candidates en utilisant les séquences d'acides aminés valides déterminées pour remplacer le ou les AAC et ensuite en rejetant toute séquence candidate avec un AAC indéterminé ;
si le troisième ensemble de séquences candidates est constitué d'une seule séquence candidate, alors l'attribution de la séquence candidate unique dans le troisième ensemble de séquences candidates en tant qu'estimation de la séquence peptidique, sinon la sélection d'une ou de plusieurs séquences candidates dans le troisième ensemble de séquences candidates pour former un quatrième ensemble de séquences candidates d'une ou de plusieurs séquences candidates de sorte que la ou les séquences candidates dans le quatrième ensemble de séquences candidates sont plus susceptibles d'être la séquence peptidique que les séquences candidates non sélectionnées dans le troisième ensemble de séquences candidates ; et
si le quatrième ensemble de séquences candidates est constitué d'une seule séquence candidate, alors l'attribution de la séquence candidate unique dans le quatrième ensemble de séquences candidates en tant qu'estimation de la séquence peptidique, sinon l'attribution de plusieurs séquences candidates dans le quatrième ensemble de séquences candidates en tant qu'estimations de la séquence peptidique.

**4.** Procédé selon la revendication 3, dans lequel la sélection de la ou des séquences candidates à partir du premier ensemble de séquences candidates pour former le second ensemble de séquences candidates comprend :

la sélection d'une ou plusieurs séquences candidates ayant la plus grande longueur d'acides aminés consécutifs parmi les séquences candidates du premier ensemble de séquences candidates pour former un cinquième ensemble de séquences candidates ;
si le cinquième ensemble de séquences candidates est constitué d'une seule séquence candidate, l'attribution du cinquième ensemble de séquences candidates au deuxième ensemble de séquences candidates, sinon la sélection d'une ou plusieurs séquences candidates ayant le plus grand nombre d'acides aminés parmi plusieurs séquences candidates du cinquième ensemble de séquences candidates pour former un sixième ensemble de séquences candidates ;
si le sixième ensemble de séquences candidates est constitué d'une seule séquence candidate, l'attribution du sixième ensemble de séquences candidates au deuxième ensemble de séquences candidates, sinon la sélection d'une ou plusieurs séquences candidates présentant la plus petite erreur de correspondance parmi plusieurs séquences candidates du sixième ensemble de séquences candidates pour former un septième ensemble de séquences candidates ;

si le septième ensemble de séquences candidates est constitué d'une seule séquence candidate, l'attribution du septième ensemble de séquences candidates au deuxième ensemble de séquences candidates, sinon la sélection d'une ou plusieurs séquences candidates avec une valeur d'intensité moyenne la plus élevée d'acides aminés récupérés parmi plusieurs séquences candidates dans le septième ensemble de séquences candidates pour former un huitième ensemble de séquences candidates ; et

si le huitième ensemble de séquences candidates est constitué d'une seule séquence candidate, l'attribution du huitième ensemble de séquences candidates au deuxième ensemble de séquences candidates, sinon la sélection d'une ou plusieurs séquences candidates avec le plus grand nombre d'occurrences pour différents types d'ions avec différents décalages parmi plusieurs séquences candidates dans le huitième ensemble de séquences candidates pour former le deuxième ensemble de séquences candidates.

5. Procédé selon la revendication 3, dans lequel la sélection d'une ou plusieurs séquences candidates à partir du troisième ensemble de séquences candidates pour former la quatrième séquence candidate comprend :

la sélection, à partir du troisième ensemble de séquences candidates, d'une ou plusieurs séquences candidates ayant le plus grand nombre d'acides aminés parmi les séquences d'acides aminés valides déterminées pour former un neuvième ensemble de séquences candidates ; et
si le neuvième ensemble de séquences candidates est constitué d'une seule séquence candidate, l'attribution du neuvième ensemble de séquences candidates au quatrième ensemble de séquences candidates, ou la sélection, dans le neuvième ensemble de séquences candidates, d'une ou plusieurs séquences candidates présentant la plus petite erreur de correspondance parmi les séquences d'acides aminés valides déterminées pour former le quatrième ensemble de séquences candidates.

6. Procédé selon la revendication 1, dans lequel la vérification du fait que l'estimation de séquence peptidique individuelle est invalide (610) comprend la vérification d'une longueur de l'estimation de séquence peptidique individuelle par rapport à une longueur correcte prédéterminée de la séquence peptidique.

7. Procédé selon la revendication 1, dans lequel la vérification du fait que l'estimation de séquence peptidique individuelle est invalide (620) comprend la réalisation d'un contrôle d'ordre pour les acides aminés « G » et « L » dans l'estimation de séquence peptidique individuelle.

8. Procédé selon la revendication 1, dans lequel la vérification du fait que l'estimation de séquence peptidique individuelle est invalide (630) comprend l'exécution d'un contrôle d'ordre sur la base de bits de contrôle d'ordre transportés dans le peptide codé par des données.

EP 4 261 826 B1

| Symbol | $S_1$ | $S_2$ | $S_3$ | $S_4$ | $S_5$ | $S_6$ | $S_7$ | $S_8$ | $S_9$ | $S_{10}$ | $S_{11}$ | $S_{12}$ | $S_{13}$ | $S_{14}$ | $S_{15}$ | $S_{16}$ | $S_{17}$ |
|---|---|---|---|---|---|---|---|---|---|---|---|---|---|---|---|---|---|
| Seq #1 | c | c | c | c | $Q_{1,2}$ | $A_1$ | $A_2$ | $A_3$ | $A_4$ | c | c | c | c | c | c | c | c |
| | c | c | c | c | $Q_{2,3}$ | | | | | c | c | c | c | c | c | c | c |
| | c | c | c | c | $Q_{1,1}$ | | | | | c | c | c | c | c | c | c | c |
| . . . | . . . | . . . | . . . | . . . | . . . | . . . | . . . | . . . | . . . | . . . | . . . | . . . | . . . | . . . | . . . | . . . | . . . |
| #4095 | c | c | c | c | $Q_{1,2}$ | $A_1$ | $A_2$ | $A_3$ | $A_4$ | c | c | c | c | c | c | c | c |
| | c | c | c | c | $Q_{2,3}$ | | | | | c | c | c | c | c | c | c | c |
| | c | c | c | c | $Q_{1,1}$ | | | | | c | c | c | c | c | c | c | c |

# FIG. 1

FIG. 2

| | c | c | c | c | $Q_{1,2}$ | | | | | c | c | c | c | c | c | | | | |
|---|---|---|---|---|---|---|---|---|---|---|---|---|---|---|---|---|---|---|---|
| #4091 | c | c | c | c | $Q_{2,3}$ | $A_1$ | $A_2$ | $A_3$ | $A_4$ | c | c | c | c | c | c | | | | |
| | c | c | c | c | $Q_{L'-1,L'}$ | | | | | c | c | c | c | c | c | | | | |
| | c | c | c | c | $Q_{1,2}$ | | | | | c | c | c | c | c | c | c | | | |
| #4092 | c | c | c | c | $Q_{2,3}$ | $A_1$ | $A_2$ | $A_3$ | $A_4$ | c | c | c | c | c | c | c | | | |
| | c | c | c | c | $Q_{L'-1,L'}$ | | | | | c | c | c | c | c | c | c | | | |
| | c | c | c | c | $Q_{1,2}$ | | | | | c | c | c | c | c | c | c | c | | |
| #4093 | c | c | c | c | $Q_{2,3}$ | $A_1$ | $A_2$ | $A_3$ | $A_4$ | c | c | c | c | c | c | c | c | | |
| | c | c | c | c | $Q_{L'-1,L'}$ | | | | | c | c | c | c | c | c | c | c | | |
| | c | c | c | c | $Q_{1,2}$ | | | | | c | c | c | c | c | c | c | c | c | |
| #4094 | c | c | c | c | $Q_{2,3}$ | $A_1$ | $A_2$ | $A_3$ | $A_4$ | c | c | c | c | c | c | c | c | c | |
| | c | c | c | c | $Q_{L'-1,L'}$ | | | | | c | c | c | c | c | c | c | c | c | |
| | c | c | c | c | $Q_{1,2}$ | | | | | c | c | c | c | c | c | c | c | c | c |
| #4095 | c | c | c | c | $Q_{2,3}$ | $A_1$ | $A_2$ | $A_3$ | $A_4$ | c | c | c | c | c | c | c | c | c | c |
| | c | c | c | c | $Q_{L'-1,L'}$ | | | | | c | c | c | c | c | c | c | c | c | c |

## FIG. 2 (CONT.)

FIG. 3

Stage 1

- 110 — Preprocess raw data of experimental spectrum to yield preprocessed data.
- 120 — Find valid paths (candidate sequences) by using preprocessed data. $n = 1$

$n > 1$

130

- 131 — Select sequences with longest length of consecutive amino acids. $n = 1$

$n > 1$

- 132 — Select sequences with largest number of amino acids. $n = 1$

$n > 1$

- 133 — Select sequences with minimum match error. $n = 1$

$n > 1$

- 134 — Select sequences with largest average intensity of amino acids. $n = 1$

$n > 1$

- 135 — Select sequences with maximum no. of occurrence of different ion types. $n = 1$

130
140 — AACs exist? — No

Yes

Stage 2

- 141 — Find valid paths in AACs by using raw data. $n = 1$

$n > 1$

- 142 — Select sequences with largest number of amino acids retrieved in AAC. $n = 1$

$n > 1$

- 143 — Select sequences with minimum match error of AAC.

100

Output candidates

$$m_{b_1} = (m_{\text{Ngroup}} - m_{\text{H}}) = 0$$

$$m_1 = m_{\text{head}}$$

$$m_{b_2} = m_{\text{head}}$$

$$m_{b_2} = m_{\text{head}} + \Delta_1$$

$$m_2$$

$$m_{b_3} = m_{b_2} + (m_2 + \Delta_2)$$

$$m_{b_3} = m_{b_2} + m_2$$

$$m_3$$

$$m_{b_3} = s_1 + s_2 + \Delta_{1,2}$$

$$m_{b_4} = \sum_{j=1}^{3} (m_j + \Delta_j)$$

$$m_{b_4} = m_{b_3} + m_3$$

$$m_{b_i}$$

$$m_{b_i} = \sum_{j=i-l}^{i-1} m_j$$

$$m_{b_{i+1}}$$

Path 2    Path 1

$$m_{b_{i+2}}$$

**AAC**

$$m_{b_{i+3}}$$

$$m_{b_{i+l}} = m_{b_i} + \sum_{j=i}^{i+l-1} (m_j + \Delta_j)$$

$$m_{b_{i+4}} = m_{b_i} + \sum_{j=i}^{i+3} m_j$$

**(a)**    **(b)**    **(c)**    **(d)**

$$m_{b_N} = M - m_{\text{H}} - m_{\text{Cgroup}} - m_{\text{tail}}$$

$$m_N = m_{\text{tail}}$$

$$m_{b_{N+1}} = M - m_{\text{H}} - m_{\text{Cgroup}}$$

$$m_{\text{Cgroup}}$$

**200**

$$m_{b_{N+2}} = M = \sum_{j=1}^{N} m_j + m_{\text{Ngroup}} + m_{\text{Cgroup}}$$

# FIG. 4

302 — Preprocess raw data of to yield preprocessed data.

304 — Sort intensities of preprocessed data; set $J = 1$ and $i = 1$; select masses of y-ions if the charge is 2, or masses of y- and b-ions if the charge is 3; set mass of peptide as $M = M_{out}$ or $M_{out} - 1.007276Da$.

306 — Set start point as mass with $J$th highest intensity.

308 — Set $W = w_i$. Select masses corr. to $W$ higher-ranking intensities.

310 — Find highest-intensity-based tag.

312 — Find prefix with max. AAC length of $L_{p1}$ for charge 2 and $L_{p1}$ & $L_{p2}$ for charge 3.

Success

314 — Find suffix with max. AAC length of $L_{s1}$ for charge 2 and $L_{s1}$ & $L_{s2}$ for charge 3.

Success

316 — Combine prefixes, tags and suffixes to give candidate sequences.

318 — Select and refine candidates by the steps 130 and 140.

Output candidates

332 — $J \leftarrow J + 1$

$i = 1$

322 — $i \leftarrow i + 1$

No

324 — $i = V$?

Yes

Fail

Fail

334 — $J = J_{max}$?

No

Yes

Fail

300

**FIG. 5**

EP 4 261 826 B1

**Highest-intensity-based tag**

head of the path | head of the tag | mass with the highest intensity | tail of the tag | tail of the path

Prefix | Tag | Suffix

# FIG. 6

Find the prefix by using
the maximum length of AACs $L_{p1}$

Failure

Success

Find the prefix by using
the maximum length of AACs $L_{p2}$

Success

Find the suffix by using
the maximum length of AACs $L_{s1}$

Failure

Success

Find the suffix by using
the maximum length of AACs $L_{s2}$

Success

500

# FIG. 7

Find the prefix by using
the maximum length of AACs $L_{p1}$

Success

500

Find the suffix by using
the maximum length of AACs $L_{s1}$

Success

# FIG. 8

Candidate sequences generated
by the highest-intensity-tag based sequencing

610 — Checking 1: Length checking
based on the relation between the length and the address

Success

620 — Checking 2: Order checking
for amino acids "G" and "L"

Success

630 — Checking 3: Order checking
based on the order-checking bits

**600**

Success

Output valid sequences

# FIG. 9

**FIG. 10**

FIG. 11

1210 — Divide m/z ratios of raw data into subsets each for isotopes of a fragment.

1220 — Compute monoisotopic mass of the fragment for each subset.

1230 — Compute intensity of the fragment for each subset.

1240 — Distribute the masses into mass sets of putative b-ions and of putative y-ions.

1250 — Generate the preprocessed data, including (monoisotopic) masses, intensities, etc.

1110

# FIG. 12

**REFERENCES CITED IN THE DESCRIPTION**

**Patent documents cited in the description**

- US 11315023 B2
- US 20210090688 A1